(19)

Europäisches
Patentamt
European
Patent Office
Office européen
des brevets

(11) **EP 4 729 055 A1**

(12) **EUROPEAN PATENT APPLICATION**

(43) Date of publication:
**22.04.2026 Bulletin 2026/17**

(21) Application number: **24386119.2**

(22) Date of filing: **18.10.2024**

(51) International Patent Classification (IPC):
*A61K 31/137* (2006.01)    *A61K 9/00* (2006.01)
*A61K 47/10* (2017.01)    *A61K 47/38* (2006.01)
*A61K 47/40* (2006.01)    *A61P 25/28* (2006.01)
*A61K 31/397* (2006.01)    *A61K 31/405* (2006.01)
*A61K 31/4245* (2006.01)    *A61K 31/426* (2006.01)
*A61K 31/4439* (2006.01)    *A61K 31/454* (2006.01)

(52) Cooperative Patent Classification (CPC):
**A61K 31/137; A61K 9/0043; A61K 9/7046;**
**A61K 31/397; A61K 31/405; A61K 31/4245;**
**A61K 31/426; A61K 31/4439; A61K 31/454;**
**A61P 25/28;** A61K 9/7069; A61K 47/40

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(71) Applicants:
• **The Provost, Fellows, Foundation Scholars, & the**
**other members of Board, of the College of the Holy**
**& Undiv. Trinity of Queen Elizabeth near Dublin**
**Dublin 2 (IE)**
• **National and Kapodistrian University of Athens**
**10561 Athens (GR)**

(72) Inventors:
• **Valsami, Georgia**
**15784 Athens (GR)**
• **Dev, Kumlesh**
**Dublin 2 (IE)**
• **Papakyriakopoulou, Paraskevi**
**15784 Athens (GR)**
• **Rekkas, Dimitrios**
**15784 Athens (GR)**

(74) Representative: **Yazitzoglou, Evagelia S.**
**Dr. Helen G. Papaconstantinou and Partners**
**2, Coumbari Street**
**Kolonaki**
**106 74 Athens (GR)**

(54) **TREATMENT OF DISORDERS USING SPHINGOSINE-1-PHOSPHATE RECEPTOR MODULATORS**

(57)    The present invention relates to the treatment of disorders using sphingosine-1-phosphate receptor modulators. Specifically, the present invention relates to the treatment of neurological disorders such as multiple sclerosis using sphingosine-1-phosphate receptor modulators such as fingolimod. Also disclosed are compositions for administration of the sphingosine-1-phosphate receptor modulator to a subject; methods for manufacturing such compositions; and methods of delivering the sphingosine-1-phosphate receptor modulator to a subject.

Figure 2

EP 4 729 055 A1

## Description

### Field of the invention

The present invention relates to the treatment of disorders using sphingosine-1-phosphate receptor modulators. Specifically, the present invention relates to the treatment of neurological disorders such as multiple sclerosis using sphingosine-1-phosphate receptor modulators such as fingolimod.

### Background to the invention

Multiple sclerosis (MS) is a chronic neurological disorder characterized by immune-mediated inflammation and demyelination in the central nervous system (CNS). MS can affect both brain and spinal cord, producing a wide range of symptoms. The severity of these symptoms depends on the nerve fiber damage in the CNS. Specifically, movement impairment is observed, expressed as muscle spasms, weakness or fatigue, vision problems, tingling of hands and feet, as well as cognitive problems. MS belongs to the category of incurable diseases, and the available treatments aim to reduce the number and severity of relapses while slowing the progression of the disease. These treatments include injectable (intramuscular or intravenous) or oral medicines that act as disease-modifying therapies (DMT).

Fingolimod hydrochloride (FH) is the first FDA-approved oral DMT and is a sphingosine-1-phosphate receptor modulator acting on lymphocytes, a type of white blood cells involved in the immune response. By activating these receptors, FH traps lymphocytes within lymph nodes, preventing the lymphocytes from circulating to the CNS and causing inflammation and damage to the myelin sheath that covers the nerve fibers. FH is characterized by poor water solubility and high lipophilicity, classifying FH as a class II drug, according to the Biopharmaceutics Classification System (BCS). The compound has a half-life of 6-9 days and reach the steady-state after 1-2 months of daily dosing. FH can cross the blood-brain barrier (BBB) acting directly on CNS by inhibiting the production of proinflammatory cytokines in microglia. Additionally, FH enhances the secretion of myelin basic protein, facilitating the remyelination process.

According to FH clinical pharmacokinetics, absorption after oral administration is high but notably slow, with a $T_{max}$ after 8-12 hours. The PK profile of FH can be attributed to the physicochemical properties of the compound (high lipophilicity, poor water solubility), as well as to a bidirectional conversion into an active metabolite, fingolimod phosphate. Additionally, FH is a substrate of the P-glycoprotein (P-gp) efflux transporter and has been found to reduce the activity of the transporter, thereby improving the absorption of small molecules into the brain. However, the high affinity for P-gp may reduce the absorption of FH itself, leading to re-transportation back into the intestinal lumen.

There is a need to provide new means for the treatment of disorders using sphingosine-1-phosphate receptor modulators, in particular new means for the treatment of neurological disorders such as multiple sclerosis using sphingosine-1-phosphate receptor modulators such as fingolimod.

### Summary of the invention

According to a first aspect of the present invention there is provided a sphingosine-1-phosphate receptor modulator for use in the treatment of a disorder in a subject, the use comprising nasal administration of the sphingosine-1-phosphate receptor modulator to the subject.

According to a second aspect of the present invention there is provided a method for the treatment of a disorder in a subject, the method comprising nasal administration of a sphingosine-1-phosphate receptor modulator to the subject.

According to a third aspect of the present invention there is provided use of a sphingosine-1-phosphate receptor modulator in the manufacture of a medicament for the treatment of a disorder in a subject, the treatment comprising nasal administration of the sphingosine-1-phosphate receptor modulator to the subject.

According to a fourth aspect of the present invention there is provided use of a sphingosine-1-phosphate receptor modulator in the treatment of a disorder in a subject, the use comprising nasal administration of the sphingosine-1-phosphate receptor modulator to the subject.

Optionally, the nasal administration comprises nasal inhalation.

Optionally, the nasal administration comprises topical administration to the nasal cavity.

Further optionally, the nasal administration comprises topical administration to a mucous membrane of the nasal cavity.

Preferably, the nasal administration comprises topical administration to the nasal cavity.

Optionally, the disorder is a neurological disorder.

Optionally, the disorder is multiple sclerosis.

Optionally, the disorder is relapsing multiple sclerosis.

Further optionally, the disorder is relapsing-remitting multiple sclerosis.

Optionally, the disorder is progressive multiple sclerosis.

Further optionally, the disorder is secondary progressive multiple sclerosis.

Still further optionally, the disorder is primary progressive multiple sclerosis.

Preferably, the disorder is multiple sclerosis.

Optionally, the disorder is ulcerative colitits (UC).

Optionally, the disorder is psoriasis.

Optionally, the sphingosine-1-phosphate receptor modulator is a sphingosine-1-phosphate receptor agonist.

Optionally, the sphingosine-1-phosphate receptor modulator is selected from fingolimod, ozanimod, siponimod, ponesimod, etrasimod, ceralifimod, zectivimod, and cenerimod.

Optionally, the sphingosine-1-phosphate receptor modulator is fingolimod.

Further optionally, the sphingosine-1-phosphate receptor modulator is 2-Amino-2-[2-(4-octylphenyl)ethyl]propane-1,3-diol.

Further optionally, the sphingosine-1-phosphate receptor modulator is fingolimod hydrochloride.

Preferably, the sphingosine-1-phosphate receptor modulator is fingolimod.

Optionally, the sphingosine-1-phosphate receptor modulator is a composition comprising the sphingosine-1-phosphate receptor modulator.

Further optionally, the sphingosine-1-phosphate receptor modulator is a pharmaceutically acceptable composition comprising the sphingosine-1-phosphate receptor modulator.

Preferably, the sphingosine-1-phosphate receptor modulator is a composition comprising the sphingosine-1-phosphate receptor modulator.

According to a fifth aspect of the present invention there is provided a composition for nasal administration of a sphingosine-1-phosphate receptor modulator to a subject, the composition comprising the sphingosine-1-phosphate receptor modulator.

Optionally, the composition comprises the sphingosine-1-phosphate receptor modulator in an amount of 0.05 - 0.5 %w/w relative to the weight of the composition.

Optionally, the composition comprises the sphingosine-1-phosphate receptor modulator in an amount of 0.05 - 0.5, optionally 0.1 - 0.45, optionally 0.15 - 0.4, optionally 0.2 - 0.35, optionally 0.25 - 0.3 %w/w relative to the weight of the composition.

Optionally, the composition comprises the sphingosine-1-phosphate receptor modulator in an amount of 0.05 - 0.15, optionally 0.1 %w/w relative to the weight of the composition.

Preferably, the composition comprises the sphingosine-1-phosphate receptor modulator in an amount of 0.1 % w/w relative to the weight of the composition.

Preferably, the composition comprises the fingolimod in an amount of 0.1 %w/w relative to the weight of the composition.

Optionally, the composition further comprises a film-forming polymer.

Optionally, the composition comprises the sphingosine-1-phosphate receptor modulator and a film-forming polymer.

Optionally, the film-forming polymer is a cellulose or a derivative thereof.

Further optionally, the film-forming polymer is a cellulose ether or a derivative thereof.

Still further optionally, the film-forming polymer is a methylcellulose or a derivative thereof.

Still further optionally, the film-forming polymer is a hydroxypropyl methylcellulose (HPMC).

Optionally, the film-forming polymer is a HPMC having a nominal methoxy substitution of about 22-29% and a nominal hydroxypropyl substitution of about 0-10%.

Further optionally, the film-forming polymer is a HPMC having a nominal methoxy substitution of about 29% and a nominal hydroxypropyl substitution of about 10%.

Preferably, the film-forming polymer is a hydroxypropyl methylcellulose (HPMC).

Optionally, the composition further comprises the film-forming polymer in an amount of 0.5 - 5.0 %w/w relative to the weight of the composition.

Optionally, the composition further comprises the film-forming polymer in an amount of 0.5 - 5.0, optionally 1.0 - 4.5, optionally 1.5 - 3.5, optionally 2.0 - 3.0, optionally 2.5 %w/w relative to the weight of the composition.

Optionally, the composition further comprises the film-forming polymer in an amount of 0.5 - 5.0, optionally 1.0 - 3.0, optionally 1.5 - 2.5, optionally 2.0 %w/w relative to the weight of the composition.

Preferably, the composition further comprises the film-forming polymer in an amount of 3.0 %w/w relative to the weight of the composition.

Preferably, the composition further comprises the HPMC in an amount of 3.0 %w/w relative to the weight of the composition.

Optionally, the composition excludes a permeation enhancer.

Optionally, the composition further comprises a permeation enhancer.

Optionally, the composition comprises the sphingosine-1-phosphate receptor modulator and a permeation enhancer.

Optionally, the composition comprises the sphingosine-1-phosphate receptor modulator, a film-forming polymer, and a permeation enhancer.

Optionally, the permeation enhancer is a cyclodextrin (CD) or derivative thereof.

Further optionally, the permeation enhancer is a beta-cyclodextrin (beta-CD) or derivative thereof.

Still further optionally, the permeation enhancer is a cycloheptakis-(1→4)-alpha-D-glucopyranosyl (cyclomaltoheptaose) or derivative thereof.

Still further optionally, the permeation enhancer is a methyl-beta-cyclodextrin (methyl-beta-CD).

Preferably, the permeation enhancer is a methyl-beta-cyclodextrin (methyl-beta-CD).

Optionally, the composition further comprises the permeation enhancer in an amount of 0.0 - 10.0 %w/w relative to the weight of the composition.

Optionally, the composition further comprises the permeation enhancer in an amount of 0.0 - 10.0, optionally 0.5 - 10.0, optionally 1.0 - 9.5, optionally 1.5 - 9.0, optionally 2.0 - 8.5, optionally 2.5 - 7.5, optionally 3.0 - 7.0, optionally 3.5 - 6.5, optionally 4.0 - 6.0, optionally 4.5 - 5.5, optionally 5.0 %w/w relative to the weight of the composition.

Optionally, the composition further comprises the permeation enhancer in an amount of 0.0 - 10.0, optionally 0.5 - 10.0, optionally 1.0 - 6.0, optionally 3.0 - 6.0, optionally 6.0 %w/w relative to the weight of the composition.

Preferably, the composition further comprises the permeation enhancer in an amount of 6.0 %w/w relative to the weight of the composition.

Preferably, the composition further comprises the methyl-beta-cyclodextrin (methyl-beta-CD) in an amount of 6.0 %w/w relative to the weight of the composition.

Optionally, the composition further comprises the permeation enhancer in an amount of 0.5 - 6.0, optionally 0.5 - 5.0, optionally 1.0 - 4.5, optionally 1.5 - 3.0, optionally 2.0 - 2.5 %w/w relative to the weight of the composition.

Preferably, the composition further comprises the permeation enhancer in an amount of 0.5 - 6.0 %w/w relative to the weight of the composition.

Preferably, the composition further comprises the film-forming polymer in an amount of 3.0 %w/w relative to the weight of the composition.

Optionally, the composition further consists of the film-forming polymer in an amount of 3.0 %w/w relative to the weight of the composition.

Optionally, the composition consists of the sphingosine-1-phosphate receptor modulator and the film-forming polymer in an amount of 3.0 %w/w relative to the weight of the composition.

Preferably, the composition further comprises the film-forming polymer in an amount of 3.0 %w/w relative to the weight of the composition and the permeation enhancer in an amount of 0.5 - 6.0 %w/w relative to the weight of the composition.

Optionally, the composition further consists of the film-forming polymer in an amount of 3.0 %w/w relative to the weight of the composition and the permeation enhancer in an amount of 0.5 - 6.0 %w/w relative to the weight of the composition.

Optionally, the composition consists of the sphingosine-1-phosphate receptor modulator, the film-forming polymer in an amount of 3.0 %w/w relative to the weight of the composition and the permeation enhancer in an amount of 0.5 - 6.0 %w/w relative to the weight of the composition.

Optionally, the composition further comprises the film-forming polymer in an amount of 3.0 %w/w relative to the weight of the composition and the permeation enhancer in an amount of 0.5 - 6.0 %w/w relative to the weight of the composition, and excludes a plasticizer.

Optionally, the composition comprises the sphingosine-1-phosphate receptor modulator, the film-forming polymer in an amount of 3.0 %w/w relative to the weight of the composition and the permeation enhancer in an amount of 0.5 - 6.0 %w/w relative to the weight of the composition, and excludes a plasticizer.

Preferably, the composition further consists of the film-forming polymer in an amount of 3.0 %w/w relative to the weight of the composition and the permeation enhancer in an amount of 6.0 %w/w relative to the weight of the composition.

Preferably, the composition consists of the sphingosine-1-phosphate receptor modulator, the film-forming polymer in an amount of 3.0 %w/w relative to the weight of the composition and the permeation enhancer in an amount of 6.0 %w/w relative to the weight of the composition.

Optionally, the composition excludes a plasticizer.

Optionally, the composition comprises of the film-forming polymer in an amount of 3.0 %w/w relative to the weight of the composition and excludes a permeation enhancer.

Optionally, the composition comprises of the film-forming polymer in an amount of 3.0 %w/w relative to the weight of the composition and excludes a plasticizer.

Optionally, the composition comprises of the film-forming polymer in an amount of 3.0 %w/w relative to the weight of the composition and excludes a permeation enhancer and excludes a plasticizer.

Preferably, the composition comprises of the film-forming polymer in an amount of 3.0 %w/w relative to the weight of the composition and the permeation enhancer in an amount of 6.0 %w/w relative to the weight of the composition, and excludes a plasticizer.

Optionally, the composition further comprises a plasticizer.

Optionally, the composition comprises the sphingosine-1-phosphate receptor modulator, a film-forming polymer, and a plasticizer.

Optionally, the composition comprises the sphingosine-1-phosphate receptor modulator, a film-forming polymer, a permeation enhancer, and a plasticizer.

Preferably, the composition comprises the sphingosine-1-phosphate receptor modulator, a film-forming polymer, a permeation enhancer, and a plasticizer.

Optionally, the plasticizer is a polyethylene glycol.

Optionally, the plasticizer is a poly(oxyethylene) or polyethylene oxide).

Optionally, the plasticizer is a polyethylene glycol having an average molecular weight of about 200 - 8000 Da.

Further optionally, the plasticizer is a polyethylene glycol having an average molecular weight of about 200 - 800 Da.

Further optionally, the plasticizer is a polyethylene glycol having an average molecular weight of about 200 - 800, optionally 400 - 600, optionally 400 Da.

Preferably, the plasticizer is a polyethylene glycol having an average molecular weight of about 400 Da.

Optionally, the composition further comprises the plasticizer in an amount of 0.0 - 5.0 %w/w relative to the weight of the composition.

Optionally, the composition further comprises the plasticizer in an amount of 0.0 - 5.0, optionally 1.0 - 4.5, optionally 1.5 - 3.5, optionally 2.0 - 3.0, optionally 2.5 %w/w relative to the weight of the composition.

Optionally, the composition further comprises the plasticizer in an amount of 0.0 - 5.0, optionally 1.0 - 3.0, optionally 1.5 - 2.5, optionally 2.0 %w/w relative to the weight of the composition.

Preferably, the composition further comprises the plasticizer in an amount of 0.0 %w/w relative to the weight of the composition.

Preferably, the composition further comprises the polyethylene glycol having an average molecular weight of about 400 Da in an amount of 0.0 %w/w relative to the weight of the composition.

Optionally, the composition further comprises the film-forming polymer in an amount of 0.5 - 5.0 %w/w relative to the weight of the composition and the permeation enhancer in an amount of 0.5 - 5.0 %w/w relative to the weight of the composition, and the plasticizer in an amount of 0.5 - 5.0 %w/w relative to the weight of the composition.

Further optionally, the composition comprises the film-forming polymer in an amount of 3.0 %w/w relative to the weight of the composition, and the permeation enhancer in an amount of 0.0 %w/w relative to the weight of the composition, and the plasticizer in an amount of 0.0 %w/w relative to the weight of the composition.

Still further optionally, the composition comprises the HPMC in an amount of 3.0 %w/w relative to the weight of the composition and the methyl-beta-cyclodextrin (methyl-beta-CD) in an amount of 0.0 %w/w relative to the weight of the composition, and the polyethylene glycol having an average molecular weight of about 400 Da in an amount of 0.0 %w/w relative to the weight of the composition.

Further optionally, the composition comprises the film-forming polymer in an amount of 3.0 %w/w relative to the weight of the composition and the permeation enhancer in an amount of 6.0 %w/w relative to the weight of the composition, and the plasticizer in an amount of 0.0 %w/w relative to the weight of the composition.

Still further optionally, the composition comprises the HPMC in an amount of 3.0 %w/w relative to the weight of the composition and the methyl-beta-cyclodextrin (methyl-beta-CD) in an amount of 6.0 %w/w relative to the weight of the composition, and the a polyethylene glycol having an average molecular weight of about 400 Da in an amount of 0.0 %w/w relative to the weight of the composition.

Optionally, the composition comprising the sphingosine-1-phosphate receptor modulator is a solid or semi-solid composition.

Preferably, the composition comprising the sphingosine-1-phosphate receptor modulator is a solid composition.

Optionally or additionally, the composition comprising the sphingosine-1-phosphate receptor modulator has a planar shape.

Optionally or additionally, the composition comprising the sphingosine-1-phosphate receptor modulator has a circular shape.

Further optionally or additionally, the composition comprising the sphingosine-1-phosphate receptor modulator has a disc shape.

Optionally, the composition comprising the sphingosine-1-phosphate receptor modulator has a diameter of 0.5-20 mm.

Further optionally, the composition comprising the sphingosine-1-phosphate receptor modulator has a diameter of 0.5-20, optionally 1-20, optionally 2-19, optionally 3-18, optionally 4-17, optionally 5-16, optionally 6-15, optionally 7-14, optionally 8-13, optionally 9-12, optionally 10-11 mm.

Still further optionally, the composition comprising the sphingosine-1-phosphate receptor modulator has a diameter of 0.5-20, optionally 1-20, optionally 1-14, optionally 2-13, optionally 3-12, optionally 4-11, optionally 5-10, optionally 6-9, optionally 7-8 mm.

Optionally, the composition comprising the sphingosine-1-phosphate receptor modulator has a cylindrical shape.

Further optionally, the composition comprising the sphingosine-1-phosphate receptor modulator has a circular cylindrical shape.

Still further optionally, the composition comprising the sphingosine-1-phosphate receptor modulator has a right circular cylindrical shape.

Optionally or additionally, the composition comprising the sphingosine-1-phosphate receptor modulator has an area of 0.05 - 1.0 cm$^2$.

Further optionally or additionally, the composition comprising the sphingosine-1-phosphate receptor modulator has a surface area of 0.05 - 1.0 cm$^2$.

Still further optionally or additionally, the composition comprising the sphingosine-1-phosphate receptor modulator has an external surface area of 0.05 - 1.0 cm$^2$.

Optionally or additionally, the composition comprising the sphingosine-1-phosphate receptor modulator has an area of 0.05 - 1.0, optionally 0.08 - 1.0, optionally 1 - 1.0, optionally 0.2 - 0.9, optionally 0.3 - 0.8, optionally 0.4 - 0.7, optionally 0.5 - 0.6 cm$^2$.

Further optionally or additionally, the composition comprising the sphingosine-1-phosphate receptor modulator has an area of 0.05 - 0.8, optionally 0.08 - 1.0, optionally 0.2 - 0.7, optionally 0.3 - 0.6, optionally 0.4 - 0.5, optionally 0.4 cm$^2$.

Still further optionally or additionally, the composition comprising the sphingosine-1-phosphate receptor modulator has an area of 0.05 - 1.0, optionally 0.06 - 0.09, optionally 0.07 - 0.08 cm$^2$.

Optionally or additionally, the composition comprising the sphingosine-1-phosphate receptor modulator has an area of 0.4 cm$^2$.

Optionally or additionally, the composition comprising the sphingosine-1-phosphate receptor modulator has an area of 0.08 cm$^2$.

Optionally or additionally, the composition comprising the sphingosine-1-phosphate receptor modulator has a weight of 0.05 - 7.0 mg.

Optionally or additionally, the composition comprising the sphingosine-1-phosphate receptor modulator has a weight of 0.05 - 7.0, optionally 0.1 - 7.0, optionally 0.5 - 7.0, optionally 1.0 - 6.5, optionally 1.5 - 6.0, optionally 2.0 - 5.5, optionally 2.5 - 5.0, optionally 3.0 - 4.5, optionally 3.5 - 4.0 mg.

Preferably, the composition comprising the sphingosine-1-phosphate receptor modulator has a weight of 3 mg.

Optionally or additionally, the composition comprising the sphingosine-1-phosphate receptor modulator has a weight of 0.05 - 0.25, optionally 0.1 - 0.2, optionally 0.15 - 0.19, optionally 0.16 - 0.17, optionally 0.17 mg.

Preferably, the composition comprising the sphingosine-1-phosphate receptor modulator has a weight of 0.17 mg.

Optionally, nasal administration comprises nasal administration of a therapeutically effective amount of the sphingosine-1-phosphate receptor modulator to the subject.

Optionally, nasal administration comprises nasal administration of the sphingosine-1-phosphate receptor modulator to the subject in an amount of 0.1 - 0.5 mg/kg.

Optionally, nasal administration comprises nasal administration of the sphingosine-1-phosphate receptor modulator to the subject in an amount of 0.1 - 0.5, optionally 0.15 - 0.45, optionally 0.2 - 0.4, optionally 0.25 - 0.35, optionally 0.2 mg/kg.

Optionally, nasal administration comprises nasal administration of the sphingosine-1-phosphate receptor modulator to the subject in an amount of 0.1 - 0.5, optionally 0.2 - 0.5, optionally 0.3 - 0.5, optionally 0.4 mg/kg.

Preferably, nasal administration comprises nasal administration of the sphingosine-1-phosphate receptor modulator to the subject in an amount of 0.4 mg/kg.

Optionally, the nasal administration comprises topical administration to the nasal cavity.

Optionally, the nasal administration is conducted for at least 5 mins.

Optionally, the nasal administration is conducted for at least 5, optionally at least 10, optionally at least 15, optionally at least 20, optionally at least 25, optionally at least 30, optionally at least 35, optionally at least 40, optionally at least 45, optionally at least 50, optionally at least 55, optionally at least 60 mins.

Optionally, the nasal administration is conducted for at least 1 hour.

Optionally, the nasal administration is conducted for at least 1, optionally at least 2, optionally at least 4, optionally at least 6, optionally at least 8, optionally at least 12, optionally at least 24, optionally at least 36, optionally at least 48 hours.

Optionally, the nasal administration further comprises oral administration of a sphingosine-1-phosphate receptor modulator.

Optionally, the nasal administration further comprises concomitant or sequential oral administration of a sphingosine-1-phosphate receptor modulator.

According to a sixth aspect of the present invention there is provided a method for manufacture of a composition for nasal administration of a sphingosine-1-phosphate receptor modulator to a subject, the method comprising providing the sphingosine-1-phosphate receptor modulator and a film-forming polymer.

Optionally, the method comprises providing the film-forming polymer and adding the sphingosine-1-phosphate receptor modulator to the film-forming polymer.

Optionally, the method comprises adding the sphingosine-1-phosphate receptor modulator in an amount of 0.05 - 0.5 %w/w relative to the weight of the composition.

Optionally, the method comprises adding the sphingosine-1-phosphate receptor modulator in an amount of 0.05 - 0.5, optionally 0.1 - 0.45, optionally 0.15 - 0.4, optionally 0.2 - 0.35, optionally 0.25 - 0.3 %w/w relative to the weight of the composition.

Optionally, the method comprises adding the sphingosine-1-phosphate receptor modulator in an amount of 0.05 - 0.15, optionally 0.1 %w/w relative to the weight of the composition.

Preferably, the method comprises adding the sphingosine-1-phosphate receptor modulator in an amount of 0.1 %w/w relative to the weight of the composition.

Preferably, the method comprises adding the fingolimod in an amount of 0.1 %w/w relative to the weight of the composition.

Optionally, the film-forming polymer is a cellulose or a derivative thereof.

Further optionally, the film-forming polymer is a cellulose ether or a derivative thereof.

Still further optionally, the film-forming polymer is a methylcellulose or a derivative thereof.

Still further optionally, the film-forming polymer is a hydroxypropyl methylcellulose (HPMC).

Optionally, the film-forming polymer is a HPMC having a nominal methoxy substitution of about 22-29% and a nominal hydroxypropyl substitution of about 0-10%.

Further optionally, the film-forming polymer is a HPMC having a nominal methoxy substitution of about 29% and a nominal hydroxypropyl substitution of about 10%.

Preferably, the film-forming polymer is a hydroxypropyl methylcellulose (HPMC).

Optionally, the method comprises providing the film-forming polymer in an amount of 0.5 - 5.0 %w/w relative to the weight of the composition.

Optionally, the method comprises providing the film-forming polymer in an amount of 0.5 - 5.0, optionally 1.0 - 4.5, optionally 1.5 - 3.5, optionally 2.0 - 3.0, optionally 2.5 %w/w relative to the weight of the composition.

Optionally, the method comprises providing the film-forming polymer in an amount of 0.5 - 5.0, optionally 1.0 - 3.0, optionally 1.5 - 2.5, optionally 2.0 %w/w relative to the weight of the composition.

Preferably, the method comprises providing the film-forming polymer in an amount of 3.0 %w/w relative to the weight of the composition.

Preferably, the method comprises providing the HPMC in an amount of 3.0 %w/w relative to the weight of the composition.

Optionally, the method excludes providing a permeation enhancer.

Optionally, the method consists of providing the sphingosine-1-phosphate receptor modulator and a film-forming polymer.

Optionally, the method further comprises providing a permeation enhancer.

Preferably, the method further comprises providing a permeation enhancer.

Optionally, the method comprises providing the film-forming polymer, adding a permeation enhancer, and adding the sphingosine-1-phosphate receptor modulator.

Optionally, the method comprises providing the film-forming polymer and adding a permeation enhancer under agitation, and adding the sphingosine-1-phosphate receptor modulator.

Further optionally, the method comprises providing the film-forming polymer and adding a permeation enhancer under stirring, and adding the sphingosine-1-phosphate receptor modulator.

Still further optionally, the method comprises providing the film-forming polymer and adding a permeation enhancer under constant stirring, and adding the sphingosine-1-phosphate receptor modulator.

Optionally, the permeation enhancer is a cyclodextrin (CD) or derivative thereof.

Further optionally, the permeation enhancer is a beta-cyclodextrin (beta-CD) or derivative thereof.

Still further optionally, the permeation enhancer is a cycloheptakis-(1→4)-alpha-D-glucopyranosyl (cyclomaltoheptaose) or derivative thereof.

Still further optionally, the permeation enhancer is a methyl-beta-cyclodextrin (methyl-beta-CD).

Optionally, the method comprises adding the permeation enhancer in an amount of 0.5 - 10.0 %w/w relative to the weight of the composition.

Optionally, the method comprises adding the permeation enhancer in an amount of 0.5 - 10.0, optionally 1.0 - 9.5, optionally 1.5 - 9.0, optionally 2.0 - 8.5, optionally 2.5 - 7.5, optionally 3.0 - 7.0, optionally 3.5 - 6.5, optionally 4.0 - 6.0, optionally 4.5 - 5.5, optionally 5.0 %w/w relative to the weight of the composition.

Optionally, the method comprises adding the permeation enhancer in an amount of 0.5 - 10.0, optionally 1.0 - 6.0, optionally 3.0 - 6.0, optionally 6.0 %w/w relative to the weight of the composition.

Preferably, the method comprises providing the permeation enhancer in an amount of 6.0 %w/w relative to the weight of the composition.

Preferably, the method comprises providing the methyl-beta-cyclodextrin (methyl-beta-CD) in an amount of 6.0 %w/w relative to the weight of the composition.

Optionally, the method excludes providing a plasticizer.

Optionally, the method consists of providing the sphingosine-1-phosphate receptor modulator and a film-forming polymer and a permeation enhancer.

Optionally, the method further comprises providing a plasticizer.

Optionally, the method comprises providing the film-forming polymer, adding a plasticizer, and adding the sphingosine-1-phosphate receptor modulator.

Optionally, the method comprises providing the film-forming polymer, adding a permeation enhancer, adding a plasticizer, and adding the sphingosine-1-phosphate receptor modulator.

Optionally, the plasticizer is a polyethylene glycol.

Optionally, the plasticizer is a poly(oxyethylene) or poly(ethylene oxide).

Optionally, the plasticizer is a polyethylene glycol having an average molecular weight of about 200 - 8000 Da.

Further optionally, the plasticizer is a polyethylene glycol having an average molecular weight of about 200 - 800 Da.

Further optionally, the plasticizer is a polyethylene glycol having an average molecular weight of about 200 - 800, optionally 400 - 600, optionally 400 Da.

Preferably, the plasticizer is a polyethylene glycol having an average molecular weight of about 400 Da.

Optionally, the method comprises providing the plasticizer in an amount of 0.5 - 5.0 %w/w relative to the weight of the composition.

Optionally, the method comprises providing the plasticizer in an amount of 0.5 - 5.0, optionally 1.0 - 4.5, optionally 1.5 - 3.5, optionally 2.0 - 3.0, optionally 2.5 %w/w relative to the weight of the composition.

Optionally, the method comprises providing the plasticizer in an amount of 0.5 - 5.0, optionally 1.0 - 3.0, optionally 1.5 - 2.5, optionally 2.0 %w/w relative to the weight of the composition.

Preferably, the method comprises providing the plasticizer in an amount of 1.5 %w/w relative to the weight of the composition.

Preferably, the method comprises providing the polyethylene glycol having an average molecular weight of about 400 Da in an amount of 1.5 %w/w relative to the weight of the composition.

Optionally, the method comprises providing step comprises providing a solution of the film-forming polymer, the permeation enhancer, and/or the plasticizer, prior to adding the sphingosine-1-phosphate receptor modulator.

Further optionally, the method comprises providing step comprises providing an aqueous solution of the film-forming polymer, the permeation enhancer, and/or the plasticizer, prior to adding the sphingosine-1-phosphate receptor modulator.

Still further optionally, the method comprises providing step comprises providing a solution of the film-forming polymer, prior to adding the permeation enhancer and/or the plasticizer, and prior to adding the sphingosine-1-phosphate receptor modulator.

Still further optionally, the method comprises providing step comprises providing an aqueous solution of the film-forming polymer, prior to adding the permeation enhancer and/or the plasticizer, and prior to adding the sphingosine-1-phosphate receptor modulator.

Optionally, the method further comprises the step of shaping the composition.

Optionally, the method further comprises the step of dehydrating the composition.

Optionally, the method further comprises the step of dehydrating the shaped composition.

Optionally, the dehydrating step is conducted for at least 1 hour.

Optionally, the dehydrating step is conducted for at least 1, optionally at least 2, optionally at least 4, optionally at least 6, optionally at least 8, optionally at least 12, optionally at least 24, optionally at least 36, optionally at least 48 hours.

Preferably, the dehydrating step is conducted for at least 24 hours.

Optionally, the dehydrating step is conducted at a temperature of at least 20 °C.

Optionally, the dehydrating step is conducted at a temperature of at least 20, optionally at least 21, optionally at least 22, optionally at least 23, optionally at least 24, optionally at least 25, optionally at least 26, optionally at least 27 °C.

Preferably, the dehydrating step is conducted at a temperature of at least 25 °C.

According to a seventh aspect of the present invention there is provided a method of delivering a sphingosine-1-phosphate receptor modulator, the method comprising providing the sphingosine-1-phosphate receptor modulator and a film-forming polymer.

Optionally, the method is a method of controlling delivering a sphingosine-1-phosphate receptor modulator.

Optionally, the method is a method of altering delivery of a sphingosine-1-phosphate receptor modulator.

Further optionally, the method is a method of altering delivery with respect to time of a sphingosine-1-phosphate receptor modulator.

Optionally, the method is a method of increasing or decreasing delivery a sphingosine-1-phosphate receptor modulator.

Further optionally, the method is a method of increasing or decreasing delivery with respect to time of a sphingosine-1-phosphate receptor modulator.

According to an eighth aspect of the present invention there is provided use of a film-forming polymer in delivering a sphingosine-1-phosphate receptor modulator.

Optionally, the use is method is use of a film-forming polymer in controlling delivery of a sphingosine-1-phosphate receptor modulator.

Optionally, the use is method is use of a film-forming polymer in altering delivery of a sphingosine-1-phosphate receptor modulator.

Further optionally, the use is use of a film-forming polymer in altering delivery with respect to time of a sphingosine-1-phosphate receptor modulator.

Optionally, the use is method is use of a film-forming polymer in increasing or decreasing delivery of a sphingosine-1-phosphate receptor modulator.

Further optionally, the use is use of a film-forming polymer in increasing or decreasing delivery with respect to time of a sphingosine-1-phosphate receptor modulator.

Optionally, the film-forming polymer is a cellulose or a derivative thereof.

Further optionally, the film-forming polymer is a cellulose ether or a derivative thereof.

Still further optionally, the film-forming polymer is a methylcellulose or a derivative thereof.

Still further optionally, the film-forming polymer is a hydroxypropyl methylcellulose (HPMC).

Optionally, the film-forming polymer is a HPMC having a nominal methoxy substitution of about 22-29% and a nominal Hydhoxypropoxyl substitution of about 0-10%.

Further optionally, the film-forming polymer is a HPMC having a nominal methoxy substitution of about 29% and a nominal Hydhoxypropoxyl substitution of about 10%.

Preferably, the film-forming polymer is a hydroxypropyl methylcellulose (HPMC).

Optionally, the increasing delivery with respect to time of a sphingosine-1-phosphate receptor modulator comprises increasing the amount of film-forming polymer.

Optionally, the decreasing delivery with respect to time of a sphingosine-1-phosphate receptor modulator comprises decreasing the amount of film-forming polymer.

## Brief description of the drawings

The present invention will now be described with reference to the following non-limiting examples and the accompanying drawings in which:

**Figure 1** illustrates permeation profiles through regenerated cellulose membrane for formulations F1-F11 in comparison to the FH solution, expressed as % of loading dose (mean $\pm$ SD, n = 3);

**Figure 2** illustrates permeation profiles through rabbit nasal mucosa for formulations F1-F11, expressed as % of loading dose (mean $\pm$ SE, n = 3);

**Figure 3** illustrates serum concentration-time profiles of FH administered intranasally, as nasal film F3 (■) and F4 (●), compared to oral administration of FH solution (▲), via gavage method, wherein the data are presented as mean $\pm$ SE, n=3-6 for IN groups, n=3-5 for PO group;

**Figure 4** illustrates total brain concentration-time profiles of FH administered intranasally, as nasal film F3 (■) and F4 (●), compared to oral administration of FH solution (·), via gavage method, wherein the data are presented as mean $\pm$ SE, n=3-6 for IN groups, n=3-5 for PO group;

**Figure 5** illustrates olfactory bulb concentration-time profiles of FH administered intranasally, as nasal film F3 (■) and F4 (●), wherein the data are presented as mean $\pm$ SE, n=3-6;

**Figure 6** illustrates a representative chromatogram of LLOQ sample (0.3 $\mu$g/mL) ($\lambda$=220 nm, t=4.2 min);

**Figure 7** illustrates a representative chromatogram of higher concentration working standard (5 $\mu$g/mL) ($\lambda$=220 nm, t=4.0 min);

**Figure 8** illustrates a calibration curve of FH in the range of 0.3 - 5 $\mu$g/mL;

**Figure 9** illustrates nasal film storage stability results, at 25 °C, presented as relative to day 0 % mean FH amount $\pm$ SD (n=3); .

**Figure 10** illustrates a FH solubility study at pH 5.6, demonstrating the effect of (A) Me-$\beta$-CD (1, 3, 6 % w/w), and HPMC E50 (1, 3 % w/w) in presence of (B) 1% Me-$\beta$-CD, (C) 3% Me-$\beta$-CD, and (D) 6% Me-$\beta$-CD [***p<0.0001, NS, not significant, (A) p=0.096, (B) p=0.200, (C) p=1.00, (D) p=0.100, n=3];

**Figure 11** illustrates Pareto charts of response Y1 (FH permeated as % of loading dose, left panel) and response Y2 (fondling times, right panel);

**Figure 12** illustrates the effect of an interaction of HPMC E50-Me-$\beta$-CD (AC), when PEG 400 was kept constant at the low level (0% w/w), on the permeation of FH across the rabbit nasal mucosa barrier, represented by the (A) 3D surface plot and (B) Interaction plot;

**Figure 13** illustrates the effect of an interaction of PEG 400-Me-$\beta$-CD (BC), when HPMC E50 was kept constant at the high level (3% w/w), on the permeation of FH across the rabbit nasal mucosa barrier, represented by the (A) 3D surface plot and (B) Interaction plot;

**Figure 14** illustrates Predicted vs Actual (Observed) plot of response Y1 (FH permeated as % of loading dose, left panel) and response Y2 (fondling times, right panel);

**Figure 15** illustrates the effect of an interaction of HPMC E50-Me-$\beta$-CD (AC) on the folding times, represented by the (A) 3D surface plot and (B) Interaction plot, wherein the plots were unaffected by the level of PEG 400;

**Figure 16** illustrates a representative chromatogram of blank serum sample ($\lambda$=220 nm);

**Figure 17** illustrates a representative chromatogram of blank brain sample ($\lambda$=220 nm);

**Figure 18** illustrates a representative chromatogram of ISTD (BRM, 0.53 $\mu$g /mL) sample spiked in blank serum ($\lambda$=315 nm, t=2.9min);

**Figure 19** illustrates a representative chromatogram of ISTD (BRM, 0.53 $\mu$g /mL) sample spiked in blank brain ($\lambda$=315 nm, t=2.9 min);

**Figure 20** illustrates a representative chromatogram of LLOQ sample (0.01 $\mu$g/mL) spiked in blank serum ($\lambda$=315 nm, t=7.3 min);

**Figure 21** illustrates a representative chromatogram of LLOQ sample (0.01 $\mu$g/mL) spiked in blank brain ($\lambda$=268 nm, t=7.0 min);

**Figure 22** illustrates a representative chromatogram of working standard sample (0.1 $\mu$g/mL) spiked in blank serum ($\lambda$=220 nm, t=7.3 min);

**Figure 23** illustrates a representative chromatogram of working standard sample (0.1 $\mu$g/mL) spiked in blank brain ($\lambda$=220 nm, t=4.5 min);

**Figure 24** illustrates a calibration curve of FH in the range of 0.01 - 0.15 $\mu$g/mL, produced in blank serum spiked with increased concentrations of the analyte;

**Figure 25** illustrates a calibration curve of FH in the range of 0.01 - 0.15 $\mu$g/mL, produced in blank brain spiked with increased concentrations of the analyte;

**Figure 26** illustrates a representative chromatogram of serum mouse sample ($\lambda$=220 nm, t=7.3 min);

**Figure 27** illustrates a representative chromatogram of brain mouse sample ($\lambda$=220 nm, t=7.0 min);

**Figure 28** illustrates observed (●) and predicted (green line) FH serum concentration (log scale) vs time, after F3 (A), F4 (B), and *per os* (C) administration of the nasal films and FH solution, respectively; and

**Figure 29** illustrates observed (●) and predicted (green line) FH brain concentration (log scale) vs time, after administration of the nasal films F3 (A) and F4 (B), andFH solution per os (C).

**Examples**

**Materials and methods**

Chemicals and Reagents

Fingolimod Hydrochloride (FH, MW: 307.47 g/mol) was supplied by Sigma Chemical Company (St. Louis, MO, USA), and Methyl-$\beta$-Cyclodextrin (Me-$\beta$-CD, MW: 1310 g/mol) was purchased from Fluka Chemika (Mexico City, Mexico US & Canada). Hydroxy propyl methyl cellulose (HPMC, Methocel E50 premium LV, MW: 90,000 g/mol) and Poly (Ethylene Gly-col) 400 (PEG 400) were purchased from Colorcon (Shanghai, China) and Sigma Chemical Company (St. Louis, MO, USA), respectively. Bromazepam (BRM, MW: 316.15 g/mol) was purchased from Sigma-Aldrich (St. Louis, MO, USA), and used as internal standard. Regenerated cellulose (RC) membranes with MW cut off: 1000 Da and diameter: 63 nm were acquired from Dianorm GmbH (Goslar, Germany). Potassium dihydrogen phosphate and Sodium hydroxide 1 mol/L were acquired from Merck KGaA (Darmstadt, Germany) and Chem-Lab NV (Zedelgem, Belgium), respectively. HPLC grade solvents (water, methanol, acetonitrile) and reagents were obtained from Fischer Scientific (Pittsburgh, PA, USA). Triple-deionized water from Fischer Scientific was used for all preparations.

Development and characterization of FH Nasal Films

The development of FH nasal films was based on a previously described method (Papakyriakopoulou P, Rekkas DM, Colombo G, Valsami G. Development and In Vitro-Ex Vivo Evaluation of Novel Polymeric Nasal Donepezil Films for Potential Use in Alzheimer's Disease Using Experimental Design. Pharmaceutics. 2022; 14(8):1742. doi: 10.3390/pharmaceutics14081742) using HPMC E50 as the film-forming polymer (Factor A: 1% to 3% w/w), PEG 400 (Factor B: 0% to 3% w/w) and Me-$\beta$-CD (Factor C: 0% to 6% w/w) as film plasticizer and permeation enhancer, respectively. Briefly, hot and cold method was used for HPMC dispersion in distilled water, followed by the addition of PEG 400 and Me-$\beta$-CD, under continuous magnetic stirring (600 rpm) to produce the film-forming gel. Weighed amounts of FH were added to the resulting gel to reach the concentration of 0.1% (w/w). A full factorial design (Design-Expert® v.11, Stat-Ease, Inc., Minneapolis, MN, USA) was employed resulting in 11 experiments shown in Table 1. The maximization of permeation across the nasal mucosa barrier and folding endurance were chosen as responses of the applied design. The films were formed from 50 $\mu$L of the gel placed on the top of cylindrical blisters, after 24 h dry in room temperature (25 °C). The produced films (F1-F11) were characterized in terms of the FH content (% of the theoretical FH film content, n=6) and stability over a period of 6 months (n=3). The parameters of film weight, thickness, folding endurance, percent (%) moisture loss, swelling, and mucoadhesive ability were measured in triplicate as described in the previously described method noted above. The in vitro and ex vivo diffusion experiments were performed using the Franz-type diffusion cells (Crown Glass, Somerville, MA, USA) and regenerated cellulose membranes (1000 Da MW cut-off) or rabbit nasal mucosa, respectively, as permeation barriers. The membrane pre-treatment and mucosa extraction were carried out as described in the previously described method noted above. Nasal mucosa was extracted on the day of the experiment from rabbit heads collected from a local slaughterhouse (Athens, Greece). The experiments were performed in conditions simulating the nasal cavity environment (T= 34 °C, 100 $\mu$L of phosphate buffer solution at pH 5.6 in the donor compartment). The loading amount of FH in the donor compartment was $0.10 \pm 0.01$ mg and the area of films ($0.41 \pm 0.03$ cm$^2$) covered all the available diffusion area ($0.40 \pm 0.05$ cm$^2$). FH solution (1 mg/mL) in buffer with pH 5.6 was also prepared and tested by introducing 100 $\mu$L of the solution in the donor compartment. All experiments lasted for 2 h and 0.5 mL were sampled from the receptor compartment and replaced by an equal volume of fresh buffer at specific time intervals (15, 30, 45, 60, 75, 90, 105, and 120 min). The samples were analysed by HPLC.

**Table 1.** Composition of the film-forming formulations derived from the experimental design.

| Formulation | HPMC E 50 | | PEG 400 | | Me-$\beta$-CD | |
|---|---|---|---|---|---|---|
| | % w/w | Coded Value | % w/w | Coded Value | % w/w | Coded Value |
| F$_1$ | 3 | 1 | 3 | 1 | 0 | -1 |

(continued)

| Formulation | HPMC E 50 | | PEG 400 | | Me-β-CD | |
|---|---|---|---|---|---|---|
| | % w/w | Coded Value | % w/w | Coded Value | % w/w | Coded Value |
| F_2 | 3 | 1 | 3 | 1 | 6 | 1 |
| F_3 | 3 | 1 | 0 | -1 | 0 | -1 |
| F_4 | 3 | 1 | 0 | -1 | 6 | 1 |
| F_5 | 1 | -1 | 0 | -1 | 0 | -1 |
| F_6 | 1 | -1 | 0 | -1 | 6 | 1 |
| F_7 | 2 | 0 | 1.5 | 0 | 3 | 0 |
| F_8 | 1 | -1 | 3 | 1 | 6 | 1 |
| F_9 | 1 | -1 | 3 | 1 | 0 | -1 |
| F_10 | 2 | 0 | 1.5 | 0 | 3 | 0 |
| F_11 | 2 | 0 | 1.5 | 0 | 3 | 0 |

The described method was applied for FH quantification in films. The thickness was measured with INSIZE Outside Micrometer (Suzhou New District, China, range of 0-25 mm, 0.001 mm graduation), while film endurance was assessed by folding the tested round films repeatedly until each film broke. The % moisture content was evaluated after 72h at 40 °C applying weight measurement before and after this time interval. Furthermore, the swelling test was performed adding 100 μL of phosphate buffer solution with pH 5.6 to each film, while the mucoadhesive ability was tested on rabbit nasal mucosa tissue positioned on glass slides in 60° degrees angle. For film packaging nylon PE vacuum bags were used and the films were evaluated for their appearance and the FH content for the period of 6 months. At the end of the experiment, the residual formulation in the donor compartment, as well as the amount accumulated in the membrane/-tissue were quantitatively collected and then quantified by HPLC to calculate the mass balance. Three factors at two different levels were chosen to optimize the formulation synthesis of FH nasal films and estimate the main effects and factor interactions. In particular, the three components served as independent variables (Factor A: HPMC; Factor B: PEG400; Factor C: Me-β-CD) varying between the low and high level which were coded as -1 and +1, respectively (see Table 2). The selection of the levels was based on the previously described method noted above. The permeation across the nasal mucosa barrier and the folding endurance were chosen as responses of the applied design constituting critical parameters of FH absorption and film administration into the nasal cavity. The maximization of these two responses during the optimization is requested to ensure adequate FH permeation across the biological barrier, as well as mechanical stability of the final film.

Table 2. Factors, levels, and responses of the experimental design.

| | Factors | Units | Low Level (-1) | High Level (+1) | Response 1 (Y1) | Response 2 (Y2) |
|---|---|---|---|---|---|---|
| A | HPMC E50 | %(w/w) | 1 | 3 60 min | % of the loading dose permeated after times | Folding |
| B | PEG 400 % (w/w) | 0 | 3 | % of the loading dose permeated after 60 min | Folding times | |
| C | Me-β-CD % (w/w) | 0 | 6 | % of the loading dose permeated after 60 min | Folding times | |

HPLC-PDA method for FH quantification in vitro/ex vivo samples

The analysis of in vitro and ex vivo samples was carried out on a Phenomenex reverse phase Gemini C-6 Phenyl column (150 × 4.6 mm, 5 μm particle size) connected to a precolumn C-6 (12.5 × 4.6 mm, 5 μm particle size, Phenomenex) of the same type. Mobile phase consisted of phosphate buffer: Acetonitrile (55:45) adjusted to pH 2.75 with orthophosphoric acid (80%), in isocratic mode with flow rate 1 mL/min. The analysis was performed at 25 °C, the injection volume was 40 μL. A previously described method (Souri E, Zargarpoor M, Mottaghi S, Ahmadkhaniha R, Kebriaeezadeh A. A Stability-Indicating HPLC Method for the Determination of Fingolimod in Pharmaceutical Dosage Forms. Sci Pharm. 2014; 83(1):85-93. doi: 10.3797/scipharm.1408-08) was optimized for the needs of the present work and the calibration curve samples range from 0.3 $\mu$g/mL to 5 $\mu$g/mL of FH.

Solubility study

The solubility study of the compound was performed either with or without Me-$\beta$-CD. The experiments were conducted at the temperature and pH conditions of the nasal cavity (pH = 5.6, T= 34 °C) using a thermostatic shaking bath (Unitronic orbital J.P. Selecta) with an adapted thermostatic unit. Moreover, the effect of Me-$\beta$-CD on the FH solubility was tested at the two concentration levels (low and high) of HPMC E50 (1 and 3% w/w). The CD effect was tested when added at the film forming gel of 1, 3, and 6% w/w HPMC E50 concentration. Particularly, 1.1 $\pm$ 0.075 mg of FH were weighed and transferred into vials of 2 mL. Then, 500 $\mu$L of A) phosphate buffer with pH 5.6, B) Me-$\beta$-CD solution (1, 3 or 6% in phosphate buffer with pH 5.6) or of C) the film forming gel [i) HPMC 1% - Me-$\beta$-CD 1%, ii) HPMC 3% - Me-$\beta$-CD 1%, iii) HPMC 1% - Me-$\beta$-CD 3%, iv) HPMC 3% - Me-$\beta$-CD 3%, v) HPMC 1% - Me-$\beta$-CD 6%, vi) HPMC 3% - Me-$\beta$-CD 6%] were added. The vials were shaken for 24 h (50 rpm) and then centrifuged (25 °C, 10,000 rpm, 10 min). The supernatant was injected after appropriate dilution into the HPLC-PDA system, and the FH concentration was measured.

Comparative pharmacokinetic study of intranasal and oral administration

Animals and Housing conditions

All animal experiments were performed in the animal facility of the Centre of Clinical, Experimental Surgery and Translational Research of the Biomedical Research Foundation of the Academy of Athens. The experimental protocol of the study was approved by the Veterinary Authorities of Region of Athens, Greece (Ethical approval num. 300643/Date of approval: 15-04-2021). The study was conducted in accordance with the ARRIVE guidelines and associated guidelines, EU Directive 2010/63/EU for animal experiments (https://eur-lex.europa.eu/legal-content/EN/TXT/?uri=CE LEX:32010L0063). The facility is registered as "breeding" and "experimental" facility according to the Greek Presidential Decree 56/2013, which harmonizes national legislation with the European Community Directive 2010/63 on the Protection of Animals used for Experimental and Other Scientific Purposes (European Union, 2010). C57BL/6J mice were used in the study and were housed in individually ventilated cages (Techniplast, IT-Varese) under specific pathogen-free (SPF) conditions and constant environmental conditions (12:12 h light:dark cycle, temperature 22 $\pm$ 2 °C, relative humidity 45 $\pm$ 10%). The mice were fed on irradiated pellets (4FR22CS diet, Mucedola, Milan, Italy) and had access to tap water and libitum. The cage bedding comprised corncob granules (REHOFIX®, J. Rettenmaier & Söhne Co., DE-Rosenberg). Cages and bedding were changed once-a-week. All mice in the facility were screened regularly according to a health-monitoring program, complying with the Federation of European Laboratory Animal Science Associations' recommendations.

Film preparation and sampling protocol

Films and solution preparation

A predetermined amount of gel was aspirated into a 24G catheter and let dry for 72 hours at room temperature (<25 °C). The films were prepared three days prior to administration and maintained their shape, dimensions, and drug content stability for six months, with 108 $\pm$ 4.7% of the initial content (day 0), stored in an airtight container at 25 °C. The solution was prepared on the day of the administration, to ensure the stability of the active substance.

Dosing and Sampling protocol

The IN administration of FH nasal film was performed following the method described by Balafas EG, Papakyriakopoulou PI, Kostomitsopoulos NG, Valsami GN. Intranasal Administration of a Polymeric Biodegradable Film to C57BL/6 Mice. J Am Assoc Lab Anim Sci. 2023; 62(2):179-184. doi: 10.30802/AALAS-JAALAS-22-00009. The film manufacturing process was adjusted for the purpose of administration in mice as described in Papakyriakopoulou P, Balafas E, Colombo C, Rekkas DM, Kostomitsopoulos N, Valsami G. Nose-to-Brain delivery of donepezil hydrochloride following administration of an HPMC-Me-$\beta$-CD-PEG400 nasal film in mice. J. Drug Deliv Sci Technol. 2023; 84, 104463. doi: 10.1 016/j.jddst.2023.1 04463 to produce a tube-shape cylinder able to be delivered and fit in the mouse nostril. The resulted films formed into the catheter's lumen had a length and weight of 0.85 $\pm$ 0.09 mm and 0.17$\pm$ 0.11 mg (n=5), respectively, while their external diameter measured 0.68 $\pm$ 0.012 mm. To assess the film content, FH quantification was performed in six films yielding 102 $\pm$ 2.12% of the theoretical dose. The FH solution for oral administration was prepared by dissolving weighed amount of the drug powder in water for injection (WFI), to achieve the concentration of 0.125 mg/mL. The volume of 0.2 mL was administered to each animal, via the oral gavage technique. Eight-week-old C57BL/6J mice were weighed (22.9 ± 1.9 g) and randomly divided in three groups (F3, F4, PO), each one receiving FH as described in Table 3. Mice received IN treatment with a 2.5-fold lower dose, taking advantage of NTB transport.

**Table 3.** Dosing and sampling protocol of pharmacokinetic study.

| Formulation | Dose mg/kg | Sampling time points min or h | N animals in each group |
|---|---|---|---|
| F3 F4 | 0.4 | 5, 10, 15, 30, 45, 60, 120, 240, and 360 min | 3-6 |
| Per os (PO) | 1 | 15, 30, 45, 60, 120, 240, and 360, 8, 10, 18 and 24 h | 3-5 |

IN administration was performed on anesthetized animals. For the IN administration sevoflurane anaesthesia, with minimum alveolar concentration (MAC) 2-4% in 100% oxygen, was induced using a calibrated vaporizer (Abbott Laboratories, Illinois, USA), and a 3D-printed rodent face mask was employed to ensure proper gas supply and scavenging. Mice were gently restrained, and a catheter was inserted into the nostril. A film was introduced into the nasal cavity using a grounded needle to prevent any harm or injury. The positioning of the film was confirmed by removing it from the catheter after administration. The entire process took less than 1 minute. Depending on the sampling design, at the predetermined time points, the animal was anesthetized and sacrificed to collect blood and brain samples. The collected blood samples were centrifuged (10,000 rpm, 15 min, 4 °C) to separate the serum. The brain was collected following total body perfusion as described by Gage GJ, Kipke DR, Shain W. Whole animal perfusion fixation for rodents, J Vis Exp 65. 2012; e3564. doi: 10.3791/3564 with cold PBS (Phosphate Buffered Saline, pH 7.4; KCl 0.2 g/L;NaCl 8 g/L; $Na_2HPO_4$ 1.15 g/L; $KH_2PO_4$ 0.2 g/L, pH 7.4, 10 mL/min) to remove residual blood. The procedure involved disinfecting the abdominal area, opening the chest, trimming tissue connected to the heart, and perfusing PBS through a syringe pump (PLUS SEP-12S) inserted into the heart's left ventricle. Serum, brain, and olfactory samples were stored at -70 °C until extraction and high-performance liquid chromatography (HPLC) analysis. After perfusion, the brain was dissected and weighed. In the case of the IN group, the olfactory bulb was isolated and weighed separately.

Quantitative Analysis of FH and its extraction from biological samples

FH was quantified in the prepared films and was determined in the samples of in vitro and ex vivo experiments using an HPLC-PDA Shimadzu prominence system. The analysis of in vitro and ex vivo samples was carried out applying the method of Souri E, Zargarpoor M, Mottaghi S, Ahmadkhaniha R, Kebriaeezadeh A. A Stability-Indicating HPLC Method for the Determination of Fingolimod in Pharmaceutical Dosage Forms. Sci Pharm. 2014; 83(1):85-93. doi: 10.3797/scipharm.1408-08), which was optimized for the needs of the present work. For the analyses of all the biological samples (serum, brain, olfactory) a bioanalytic method was developed and validated.

HPLC bioassay

Instrumentation and for method for FH quantification in biological samples

Chromatography was performed using a Shimadzu HPLC system (Shimadzu, Kyoto, Japan) coupled with a LC-20AD Quaternary Gradient Pump with DGU-20A5R degasser, an SIL-HTc autosampler with a temperature-controlled sample tray set at 4 °C and a CTO-20AC column oven set at 28 °C. For all biological samples chromatographic separation was carried out on a Waters reverse phase XterraTM C18 column (150 × 4.6 mm, 5 μm particle size) connected to a precolumn C-18 (12.5 × 4.6 mm, 5 μm particle size, Waters) of the same type. Bromazepam was employed as ISTD and detected at 315 nm (2 μg/mL in methanol). The calibration curve standards ranged from 0.01 μg/mL to 1 μg/mL of FH. Analysis was conducted in an isocratic elution program at a constant flow rate of 0.8 mL/min and mobile phase consisted of phosphate buffer: Acetonitrile (55:45) adjusted to pH 2.7 with phosphoric acid (80%). The LC analysis time was 10 min. The injection volume was 40 μL, while the retention time of FH was 7.3 and 7.0 min, for serum and brain tissue samples, respectively, while for ISTD 2.9 min, for all the tested samples. Detection of FH and BRM was performed at 220 and 315 nm, respectively, using an SPD-M20A PDA detector (Shimadzu, Kyoto, Japan). The DAD spectra were acquired with 4 nm resolution in the range 200-400 nm and acquisition and data analysis were performed using the LC Solution Software (Shimadzu, Kyoto, Japan). The calibration curve samples range from 0.01 μg/mL to 0.15 μg/mL of FH.

Preparation of standards

Stock and Working Standard (WS) solutions were prepared by dissolving or diluting adequate amounts of the reference materials in MeOH and mobile phase, respectively. Two different series of stock solutions were prepared separately for calibration WS and Quality Control (QC) samples. FH stock solutions (A & B) were prepared at a concentration of 50 $\mu$g/mL by dissolving 1 mg of the reference API in MeOH (20 mL). Dilutions of stock solution A were used to produce working solutions at a concentration range from 0.075 to 1.125 $\mu$g/mL, while dilutions of stock solution B were made to provide QC solutions at three different concentrations (0.01 $\mu$g/mL, 0.08 $\mu$g/mL, and 0.15 $\mu$g/mL) to investigate intra- and inter-run variations. Stock solutions were stored at -26 °C in vials, while WSs were prepared on the day of analysis. A stock solution of ISTD (BRM, 0.5 mg/mL) was prepared in MeOH and diluted with the same solvent, at a final concentration of 1 $\mu$g/mL. Matrix calibration curves and QC samples were daily prepared in blank mouse plasma and brain, spiked with 10 $\mu$L of the WS or QC solutions and 40 $\mu$L of the ISTD solution.

FH extraction from biological samples

Plasma/brain samples (25 $\mu$L) were spiked with 40 $\mu$L of the ISTD methanolic solution (1 $\mu$g /mL) and 10 $\mu$L of mobile phase, and vortex-mixed for 10 s. After centrifugation at 10,000 rpm for 10 min, at 4 °C, the supernatant was transferred into an autosampler vial and 40 $\mu$L were injected into the High-performance Liquid Chromatography with Photo Diode Array Detector (HPLC-PDA) system.

Statistical Analysis

The design space was constructed and analyzed using the Design-Expert® Software, v.11 (Stat-Ease, Inc., Minneapolis, MN, USA). Analysis of variance (ANOVA) was applied to test the chosen model and individual terms in the model. Concerning the in vitro release and ex vivo permeation experiments, data distribution was tested using the Shapiro-Wilk (S-W) normality test. Significance was set at $p < 0.05$ level and all tests were two-tailed with 95% Confidence Intervals (CI). Results are expressed as mean $\pm$ SD for the in vitro release experiments and mean $\pm$ standard error of the mean (SEM) for ex vivo permeation experiments. The permeation values were statistically compared between the different formulations and per time point within the formulation. The in vivo results are expressed as mean $\pm$ SEM and the pharmacokinetic profiles were statistically compared between the two routes of administration and per time point within the same route. Outlier detection occurred applying the Interquartile Range (IQR) using a step of $1.5 \times$ IQR. No outliers were detected. One-way ANOVA with Bonferroni post hoc test for multiple comparisons was applied, as well as Mann-Whitney nonparametric test between all group pairs. Kruskal-Wallis was performed to statistically evaluate the differences between the formulations at every time point of the experiment and post-hoc Mann-Whitney to detect individual differences. The analysis was performed using GraphPad Prism 8.0 software package (GraphPad® Software).

Non-compartmental analysis

Phoenix® 8.3.5 (Certara, Princeton, NJ, USA) was used to perform sparse sampling non-compartmental analysis (NCA) on in vivo data. Applying this methodology several pharmacokinetic parameters for serum, brain, and olfactory bulb were determined, including $AUC_{0-t}$ (the area under the concentration-time curve from time 0 to the last time point of the study), $AUC_{inf}$ (the area under the concentration-time curve extrapolated to infinity), $C_{max}$ (maximum observed concentration), $t_{max}$ (the time when $C_{max}$ is observed), $t_{1/2}$ (elimination half-life), and plasma and brain clearance, CLs and CLs. Additionally, the relative bioavailability ($F_{rel}$) of FH following the IN administration was calculated using the following equation:

$$\%F_{rel} = \frac{AUC_{inf(IN)} \times Dose_{(PO)}}{AUC_{inf(PO)} \times Dose_{(IN)}} \times 100$$

For the statistical analysis of in vivo data GraphPad Prism 8.0 software package (GraphPad® Software) was also employed.

Relative drug targeting efficiency percentage and NTB direct transport percentage indexes

In order to assess the direct transport of FH to the brain, the indexes of Drug Targeting Efficiency Percentage (DTE) and Direct Transport Percentage (DTP) from the nose to the brain can be employed. The DTE represents the relative exposure of the brain to a drug administered through the nasal route compared to oral administration. The second index, known as nose-to-brain DTP, is employed to calculate the percentage of drug that is transferred directly to brain from the nasal cavity. The equations used to calculate both indexes DTE and DTP are given below:

$$DTE = \frac{\left(\frac{AUC_{0 \to t\,(Brain)}}{AUC_{0 \to t\,(Serum)}}\right)_{in}}{\left(\frac{AUC_{0 \to t\,(Brain)}}{AUC_{0 \to t\,(Serum)}}\right)_{iv}} \times 100$$

$$DTP = \frac{\left[AUC_{0 \to t\,(Brain)}\right]_{in} - \left[AUC_{0 \to t\,(x)}\right]}{\left[AUC_{0 \to t\,(Brain)}\right]_{in}} \times 100$$

where

$$AUC_{0 \to t\,(x)}$$

is calculated by the equation:

$$AUC_{0 \to t\,(x)} = \frac{\left[AUC_{0 \to t\,(Brain)}\right]_{iv}}{\left[AUC_{0 \to t\,(Serum)}\right]_{iv}} \times \left[AUC_{0 \to t\,(Serum)}\right]_{in}$$

The DTE values can vary from 0 to $+\infty$ and the values higher than 100 indicate the superiority of nose-to-brain for brain targeting against the oral route, while DTP values can vary from $-\infty$ to 100 and any value equal to zero or lower implies that the drug is delivered to the brain only indirectly, through the BBB (Tiozzo Fasiolo L, Manniello MD, Banella S, Napoli L, Bortolotti F, Quarta E, Colombo P, Balafas E, Kostomitsopoulos N, Rekkas DM, Valsami G, Papakyriakopoulou P, Colombo G, Russo P. Flurbiprofen sodium microparticles and soft pellets for nose-to-brain delivery: Serum and brain levels in rats after nasal insufflation. Int J Pharm. 2021; 605:120827. doi: 10.1016/j.ijpharm.2021.120827).

**Example 1**

**Development and characterization of FH nasal films**

FH transparent circular nasal films were developed with mean diameter of 7.1 $\pm$ 0.21 mm and drug content of 90.2 to 107.0% (0.080 to 0.11 mg of FH, respectively) of the theoretical loading dose (observed difference less than 10%), and % standard deviations ranged from 2.5% to 5.5%. This amount corresponds to one fifth (1/5) of the recommended oral dose for adults with MS (see, for example, Gilenya, INN-fingolimod, Summary of products characteristics, EMA https:// www.ema.europa.eu/en/documents/product-information/gilenya-epar-product-information_en.pdf). The parameters of fold times, film weight, thickness, folding endurance, percent (%) moisture loss, swelling, and mucoadhesive ability were found to be within the ranges previously described by Papakyriakopoulou P, Rekkas DM, Colombo G, Valsami G. Development and In Vitro-Ex Vivo Evaluation of Novel Polymeric Nasal Donepezil Films for Potential Use in Alzheimer's Disease Using Experimental Design. Pharmaceutics. 2022; 14(8):1742. doi: 10.3390/pharmaceutics14081742. The mean FH content of all formulations ranged from 90% to 120% of the initial content (day 0).

**Example 2**

**Factorial Design of Experiments**

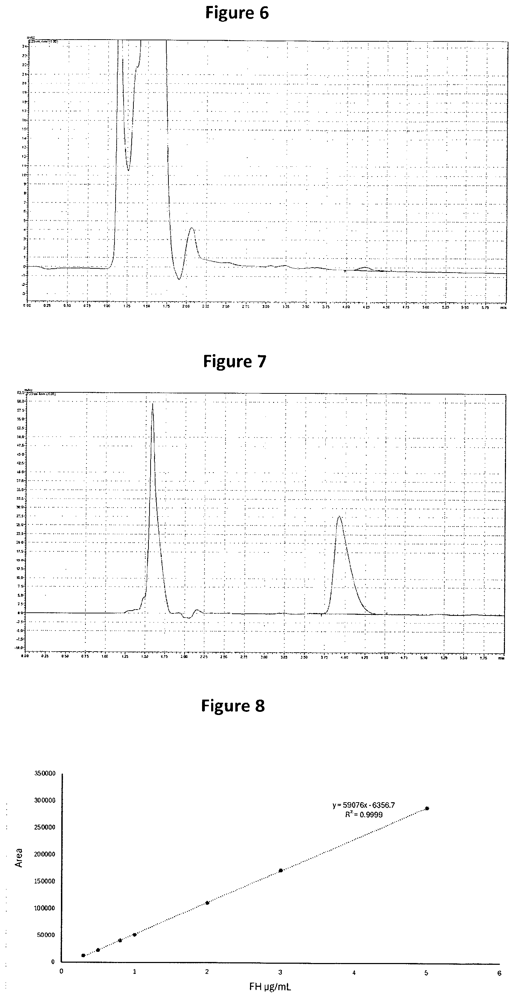

The above findings were also confirmed by the application of DoE methodology for the amount of FH permeated at 60 min ($Y_1$ response), expressed as % of the loading dose, that revealed a two-factor interaction (2FI) model ($R^2$= 0.9499, F= 114.65, p<0.0001), with no significant curvature (p=0.1360) and lack of fit (p=0.3025). To address heteroscedasticity in the data, a square root transformation was applied. The analysis of variance (ANOVA) indicated that factors A (HPMC E50) and B (PEG 400) were statistically significant (p<0.0001), while their interaction was not considered significant (p=0.2001). Furthermore, the interactions AC (HPMC E50-Me-$\beta$-CD) and BC (PEG 400- Me-$\beta$-CD) were found to be statistically significant (p<0.0001) (see Table 4 and Figure 8).

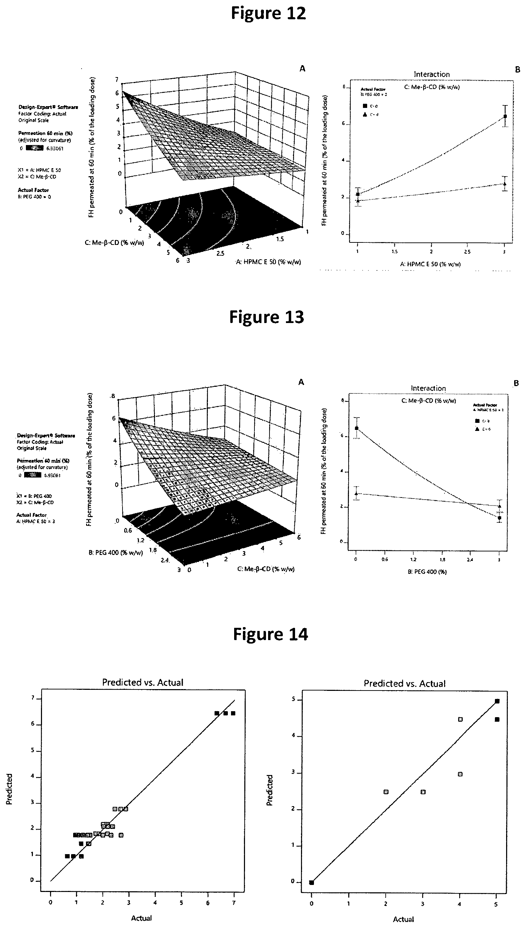

The higher amount of HPMC E50 (Factor A) in the formulation positively affects FH permeation across the biological barrier. When low-viscosity HPMC polymers co-processing with a low-solubility API, a transition of the compound in an amorphous form can be achieved, resulting in significant improvement of its solubility. The impact of HPMC E50 concentration was found to be more evident when the amount of Me-$\beta$-CD in the formulation decreases, as the high solubilizing effect of CD mask the lower positive effect of HPMC (see Figure 12). However, the comparison of F3 versus F4 ex vivo profiles indicates that the interaction of mucosa with the HPMC-Me-β-CD matrix decrease the diffusivity of FH across the biological barrier. This observation is probably related to the high concentration of Me-β-CD. In low concentrations (1% w/v), Me-β-CD can increase permeation of melatonin across EpiAirway™-100 cultures, but in higher concentrations (5 and 10% w/v) reduce drug permeation.

Regarding the BC interaction, when factor A is at its highest level (3%), the absence of Me-β-CD (low level of factor C) results in a negative, concentration-dependent effect of factor B (see Figure 13) and maximization of permeation when factor B is at its low level (0%). FH contains a tertiary amine group in its structure, allowing for its characterization as a weak base. PEG 400 can decrease the permeability of weak bases. Considering the importance of Y2 response in the optimization process, the desirability function was applied. In this regard, the factors were allowed to vary between their low and high levels, and the optimization goal was set as the maximization for both responses Y1, Y2. Furthermore, the importance of each response was rated as 5. By combining the results of the two responses, F3 was reaffirmed as the optimum formulation with a desirability score of 0.913.

**Table 4.** Independent factors for the estimated effects, F-values, and associated p-values for response 1 (% of loading dose permeation after 60 min), after the analysis of variance

| ANOVA for 2FI model | F-Value | p-Value |
| --- | --- | --- |
| Model | 114.65 | <0.0001 significant |
| A-HPMC E 50 | 151.76 | <0.0001 |
| B-PEG 400 | 189.30 | <0.0001 |
| AC | 36.11 | <0.0001 |
| BC | 81.42 | <0.0001 |

## Example 3

### Fold times of the prepared films

The results of flexibility expressed as film folding times ($Y_2$ response) were are shown in Table 5. Particularly, when factor C (Me-β-CD) is at its high level of 6% w/w, it results in a decrease in film flexibility. This reduction is particularly noticeable when plasticizer (PEG 400) is absent and when factor A (HPMC E50) is at its lower level. Specifically, the flexibility follows the pattern F6 < F4 < F2. Additionally, when factor C is at its high level, it becomes challenging to form a film that can be detached from the blister, especially when all three ingredients are presented in the formulation and the C:A ratio exceeds 2, as observed in the case of F8 (A:B:C = 1:3:6).

**Table 5.** Fold times of the prepared films

| Fold Times | Formulation |
| --- | --- |
| 0 | F6, F8 |
| 2 | F4 |
| 3 | F2 |
| 4 | F3, F7, F10, F11 |
| 5 | F1, F5, F9 |

The DoE implementation proposed a modified 2FI model ($R^2$= 0.9688, F= 289.33, p<0.0001) for $Y_2$ response. The analysis of variance (ANOVA) indicated that factors A (HPMC E50) and C (Me-β-CD) were statistically significant (p<0.0001), while their interaction was also considered significant (p<0.0001) (see Table 6).

**Table 6.** Independent factors for the estimated effects, F-values, and associated p-values for response 2 (film folding times), after the analysis of variance

| ANOVA for 2FI model | F-Value | p-Value |
| --- | --- | --- |
| Model | 289.33 | <0.0001 significant |
| A-HPMC E 50 | 56.00 | <0.0001 |
| B-PEG 400 | 686.00 | <0.0001 |

(continued)

| ANOVA for 2FI model | F-Value | p-Value |
| --- | --- | --- |
| AC | 126.00 | <0.0001 |

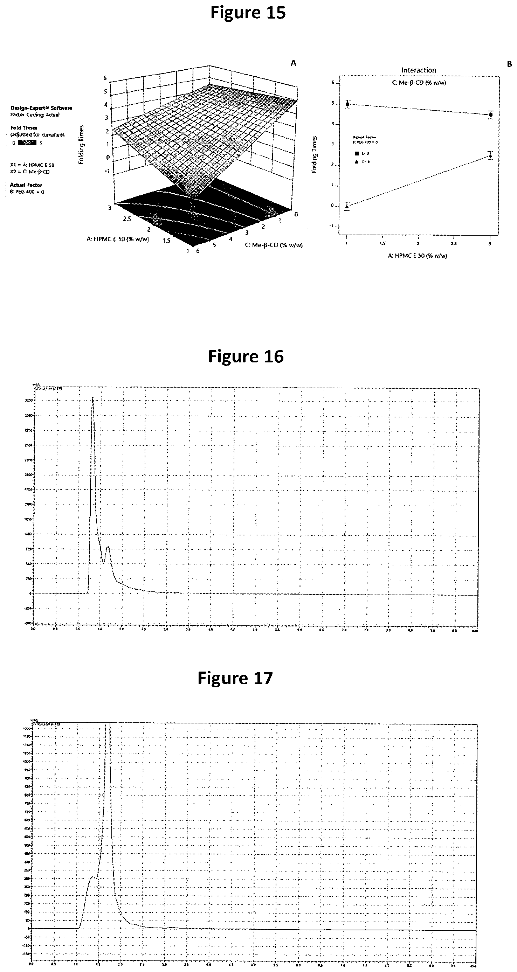

The curvature significance was found to have a non-substantial impact on the response variable as the data are adequately described by a simplified model, as also revealed by the predicted versus actual plot (see Figure 14). As revealed by the 3D surface and interaction plots (see Figure 15), when the level of factor A increases from 1% to 3%, the AC interaction improves the film flexibility, expressed as film folding times. Therefore, the negative effect of factor C is eliminated at the high level of HPMC E50 (3% w/w), where the AC interaction is more intense.

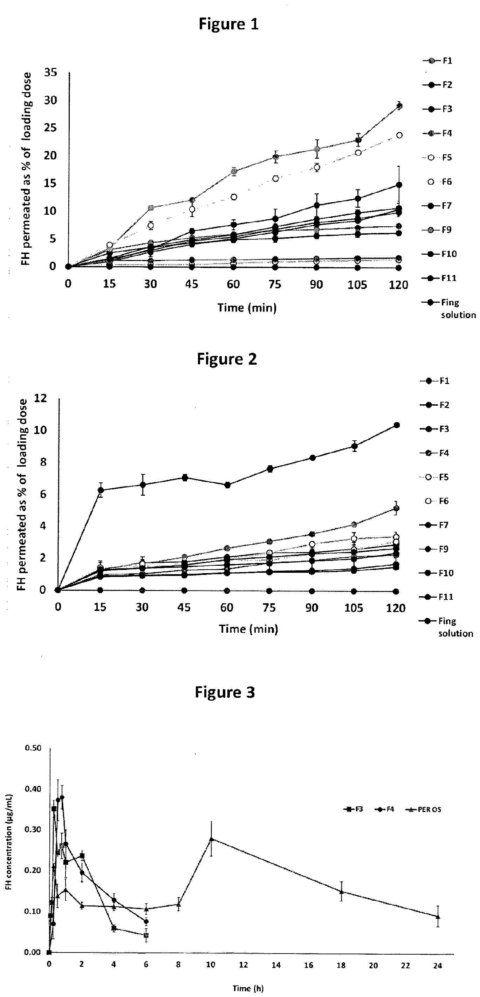

Based on the ex vivo profiles of all tested formulations, it is evident that the F3 formulation exhibits the optimal permeation profile for FH (see Figure 2). However, considering the results of the respective in vitro experiments (Figure 1), both F3 and F4 formulations were selected to proceed with the PK study and IN administration in mice. This decision aims also to investigate the impact of Me-$\beta$-CD solubilizing effect on PK parameters.

## Example 3

### Comparative pharmacokinetic study of intranasal and oral administration

F3 and F4 formulations were formed inside the lumen of a 24G catheter following the previously developed method of Balafas EG, Papakyriakopoulou PI, Kostomitsopoulos NG, Valsami GN. Intranasal Administration of a Polymeric Biodegradable Film to C57BL/6 Mice. J Am Assoc Lab Anim Sci. 2023; 62(2):179-184. doi: 10.30802/AALAS-JAALAS-22-000091. According to this method, the film was introduced into the mouse nostril, under inhaled anesthesia, shoved by a grounded needle, to avoid harm or injury during the administration.

A linear relationship was observed between the peak area or peak area ratios (FH peak area/IS peak area) and the nominal concentration of FH across both examined range (0.1 - 5 $\mu$g/mL and 0.01 - 0.15 $\mu$g/mL, respectively). The calibration curves for serum and brain samples yielded overall correlation coefficients ($R^2$) of 0.996 ($\pm$ 0.354%, RSD (%)) and 0.996 ($\pm$ 0.191%, RSD (%)), respectively. The LOD and LLOQ for FH in mouse serum were determined to be 0.0033 $\pm$ 0.00052 and 0.0099 $\pm$ 0.0016 $\mu$g/mL, respectively. For brain tissue, the LOD and LLOQ were found to be 0.004 $\pm$ 0.0003 and 0.011 $\pm$ 0.00076 $\mu$g/mL, respectively. In all cases, the back-calculated concentrations of the calibration standards fell within $\pm$ 15% of the nominal value, and within $\pm$ 20% for the LLOQ.

## Example 4

### HPLC-PDA method for FH quantification in vitro/ex vivo samples

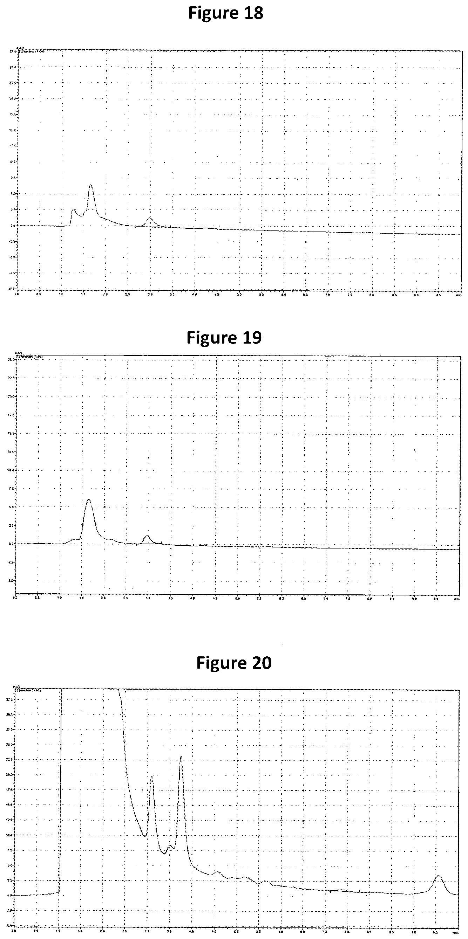

Figure 19 to Figure 24 provide further details on the validation of the HPLC method and representative chromatograms of a working standard sample (0.1 $\mu$g/mL) spiked in blank serum and brain, as well as chromatograms of serum and brain samples after IN and PO administrations. A partial validation of the method of Souri E, Zargarpoor M, Mottaghi S, Ahmadkhaniha R, Kebriaeezadeh A. A Stability-Indicating HPLC Method for the Determination of Fingolimod in Pharmaceutical Dosage Forms. Sci Pharm. 2014; 83(1):85-93. doi: 10.3797/scipharm.1408-08 was performed according to the European Medicines Agency (EMA) international guidelines on validation of analytical procedures (European Medicines Agency, Guideline on bioanalytical method validation, European Medicines Agency, 2023. Available at: https://www.ema.europa.eu/en/documents/scientific-guideline/ich-q2r2-guideline-validation-analytical-proce dures-step-5-revision-1_en.pdf). Regression and data analysis were performed by GraphPad Prism 8.0 software package (GraphPad Software).

Different mice blank serum and brain samples were analyzed individually and evaluated for the presence of potential interferences (endogenous compounds and co-medications) (see Figure 16 and Figure 17). Comparison of blank serum and brain samples spiked with the analyte and the ISTD at the expected lower limit of quantification (LLOQ) will allow examining the presence of potential interfering substances at the expected retention time of ISTD at 315 nm (see Figure 18 to Figure 21). The absence of interfering components from all the examined matrices was confirmed at the expected retention times for both FH and ISTD.

Mice blank plasma samples injected after the calibration standard at the upper LOQ did not show any evidence of carry-over contamination neither for FH nor for the ISTD.

Six sets of calibration curves, in blank serum and brain, were prepared and analyzed on different days according to the sample preparation procedure described above, consisting of seven calibration standards, a zero-blank sample (blank matrix and ISTD) and a blank matrix sample (without ISTD), for each matrix.

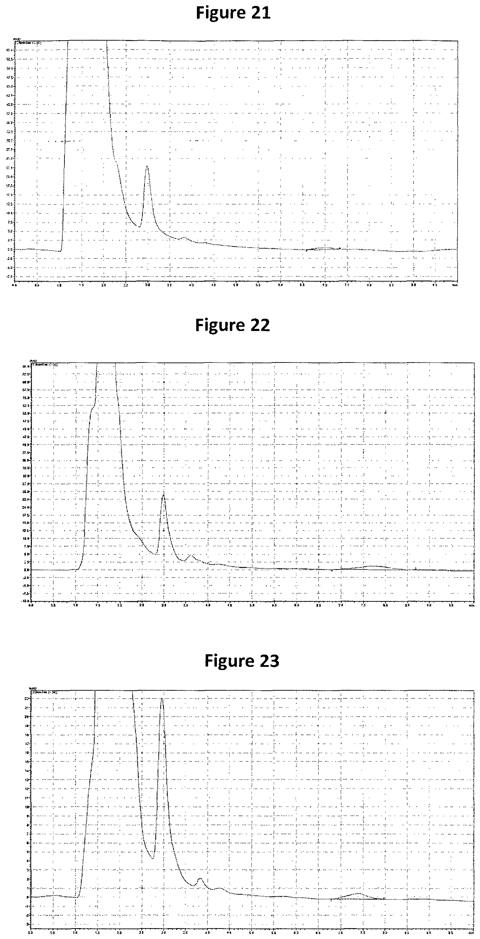

Calibration standard samples were prepared in triplicate by spiking mice blank serum or brain with FH working solutions (derived from stock solution I) within a concentration range from 0.01 to 0.15 µg/mL. Calibration curves were obtained by plotting the peak area ratios (FH peak area/ISTD peak area) versus FH nominal concentration over the examined range and fitted to the equation y = a + bx after a least-squares linear regression analysis. Representative chromatograms of the calibration curve sample with concentration 0.1 µg/mL in spiked blank serum and brain are presented in Figure 22 and Figure 18.

Representative chromatograms of the calibration curve sample with the lower (0.5 µg/mL) and the higher (7 µg/mL) concentration are presented in Figure 6 and Figure 7. A linear relationship was revealed between the FH peak area versus FH nominal concentration over the examined range (0.3 - 5 µg/mL) (Figure 8). An overall correlation coefficient (r) of 0.9999 (± 0.0197%, RSD (%)) was obtained, while at all cases, back calculated concentrations of the calibration standards were within ± 15% of the nominal value and ± 20% for LLOQ, ranging from -7.1 to 2.26 %.

A linear relationship was revealed between the peak area ratios (FH peak area/ISTD peak area) versus FH nominal concentration over the examined range (0.01 - 0.15 µg/mL) (Figures S19-20). An overall correlation coefficient (r) of 0.996 (± 0.353%, RSD (%)) and 0.996 (± 0.191%, RSD (%)) were obtained for the serum and brain sets of calibration curves, respectively. In all cases, back calculated concentrations of the calibration standards were within ± 15% of the nominal value and ± 20% for LLOQ.

Quality control (QCs) samples were prepared in triplicate derived from stock solution II to provide three different concentration levels equivalent to 0.3 µg/mL, 1 µg/mL, and 5 µg/mL and analyzed against each of the six calibration curves. The results of within-run accuracy were acceptable with Er (%) values less than 8.54% for LLOQ and not greater than 2.5% for low, medium, and high QC samples. Between-run accuracy was ranged from -6.5% to 5.9% expressed as the overall mean Er (%) for each of the examined QC concentration levels on the six analytical runs.

LOD was defined as a signal at least 3.3-fold greater than baseline noise. LOD of FH was found equivalent to 0.027 ± 0.0054 µg/mL, will LLOQ was set at 10 times the signal-to-noise (S/N) and found equal to 0.083 ± 0.016 µg/mL.

The calculated acceptance values (%) were below 15%, ranging from 8% to 14% [European Pharmacopoeia 8.0. Uniformity of Dosage Forms]. The results of content uniformity of the 11 formulations are presented in Table 7 and are expressed as % of the theoretical FH film content (mean ± SD).

**Table 7.** Film content uniformity measurements expressed as % of the theoretical FH film content [mean (SD), n=6]

| Formulation | % content | AV(%)* |
|---|---|---|
| $F_1$ | 108 (4.04) | 11 |
| $F_2$ | 111 (5.51) | 14 |
| $F_3$ | 106 (10.8) | 14 |
| $F_4$ | 112 (9.64) | 9 |
| $F_5$ | 99 (6.11) | 12 |
| $F_6$ | 100 (6.81) | 11 |
| $F_7$ | 109 (2.52) | 3 |
| $F_8$ | 106 (9.02) | 7 |
| $F_9$ | 111 (9.02) | 12 |
| $F_{10}$ | 117 (11.5) | 6 |
| $F_{11}$ | 112 (2.65) | 3 |

[0265] * AV is the Acceptance value, calculated using the equation: $AV=|M-\overline{X}|+ks$, where M is the reference value, $X$ is the mean of individual contents, and ks is the acceptability constant (Guideline on bioanalytical method validation, EMA).

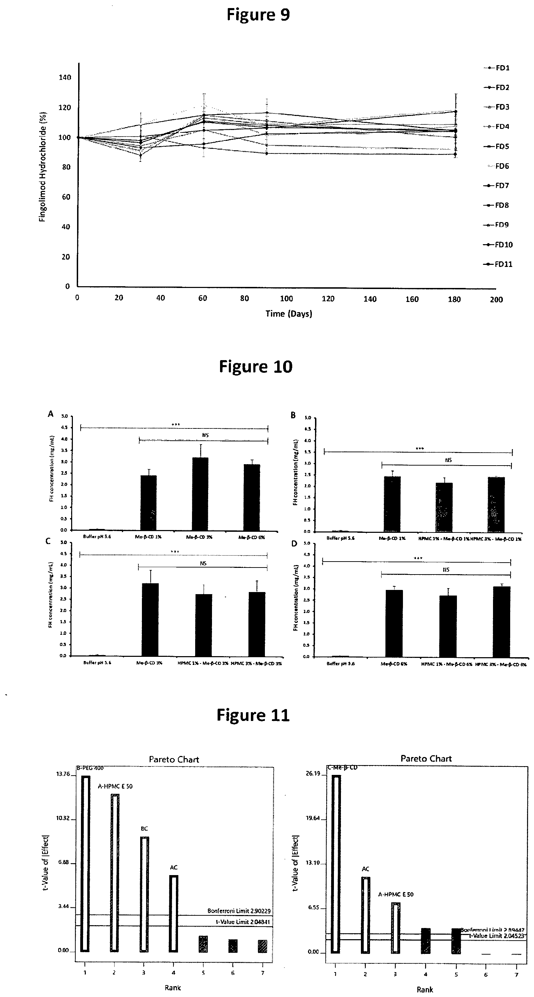

Due to the actual composition, formulation F8 did not result in the formation of a film; therefore, measurements for film thickness and the percentage (%) moisture loss were not carried out for F8. In terms of stability, the films' appearance remained unchanged after six months of storage (see Figure 9). The individual mean values of FH content (expressed as a percentage of the initial content) for each formulation, at different time points of the stability study (one, two, three, six months) are provided in Table 8.

**Table 8.** Nasal film storage stability results, at 25 °C, presented as relative to day 0 % mean FH amount ± SD (n=3)

| Formulation | Day 30 | Day 60 | Day 90 | Day 180 |
|---|---|---|---|---|
| $F_1$ | 91 ± 4.4 | 113 ± 4.05 | 110 ± 4.83 | 110 ± 7.51 |
| $F_2$ | 88 ± 3.7 | 88 ± 3.7 | 88 ± 3.7 | 88 ± 3.7 |
| $F_3$ | 91 ± 2.0 | 116 ± 4.10 | 102 ± 5.35 | 108 ± 4.73 |
| $F_4$ | 95 ± 5.6 | 105 ± 2.14 | 96 ± 3.22 | 93 ± 3.2 |

(continued)

| Formulation | Day 30 | Day 60 | Day 90 | Day 180 |
|---|---|---|---|---|
| $F_5$ | 109 ± 4.10 | 115 ± 8.74 | 117 ± 5.58 | 106 ± 11.1 |
| $F_6$ | 109 ± 7.61 | 122 ± 7.55 | 108 ± 18.2 | 120 ± 4.23 |
| $F_7$ | 98 ± 3.8 | 111 ± 3.30 | 107 ± 1.40 | 106 ± 3.30 |
| $F_8$ | 101 ± 3.32 | 105 ± 5.27 | 107 ± 3.41 | 119 ± 10.93 |
| $F_9$ | 93 ± 15 | 93 ± 5.29 | 90 ± 8.4 | 90 ± 2.1 |
| $F_{10}$ | 93 ± 2.2 | 96 ± 2.75 | 103 ± 6.06 | 105 ± 5.15 |
| $F_{11}$ | 97 ± 3.4 | 111 ± 1.36 | 109 ± 3.72 | 105 ± 6.76 |

## Example 5

### Solubility study

The solubilizing effect of Me-β-CD is evident in the results of in vitro diffusion experiments. In particular, the formulations containing 6% of Me-β-CD (F2, F4, F6) presented superior permeation profiles, whereas the permeated amount decreases as the amount of CD decreases as well (to 3% in the cases of F7, F10, and F11) (see Figure 1). This observation is attributed to the solubilizing effect of cyclodextrin, which was further investigated and confirmed in a solubility study where different concentrations of Me-β-CD (1%, 3%, and 6% w/w) were added in FH solution with concentration of 1.02 ± 0.03 mg/g in phosphate buffer solution with pH 5.6.

The FH solubility study at pH 5.6 showed that 2.32 ± 0.309 % of the loading amount was dissolved resulting in the concentration of 0.05 ± 0.01 mg/mL (see Figure 7 A-D, first column). Me-β-CD significantly (***$p<0.0001$) increased the solubilized amount of FH at all the tested concentrations (1-6%), with values of 2.43, 3.22, and 2.94 mg/mL observed in the presence of 1%, 3%, and 6% Me-β-CD, respectively (see Figure 10A). Within this range, the highest concentration value of 3.15 mg/mL value was reported for formulation F4, which contained 3%HPMC E50 and 6% Me-β-CD. Importantly, in all cases, the entire loaded amount of FH was dissolved. HPMC contribution is calculated corresponding to 4-15% increase in FH concentration values. It is important to note that no statistically significant difference was observed between the two tested concentrations of HPMC (1 and 3%).

## Example 6

### In Vitro and Ex vivo Diffusion Experiments

A negative effect of PEG 400 can be assumed when it coexists with Me-β-CD in the synthesis. In the case of F2, which includes 6% Me-β-CD and an additional 3% of PEG 400, FH diffusion was lower compared to F4, which does not include PEG 400 ($p=0.0078$). Without being bound by theory, it can be hypothesized that the FH-Me-β-CD interaction is influenced by the competition between the PEG and drug molecules, for binding to CD, as well as by the pattern of water loss from the CD molecule. The presence of HPMC can increase the solubility of highly lipophilic drugs such as fingolimod (log P=5.5). This effect is more pronounced at the high concentration of HPMC (3% w/w) as revealed by the diffusion profiles of F1 and F3 formulations. However, the contribution of HPMC is masked by the high solubilizing effect of CD, when the two excipients co-exist in the formulation. This is also evident in the similar profiles ($p=0.505$) of F4 and F6, containing 1 and 3% of HPMC E50, respectively.

The interactions between HPMC E50 and Me-β-CD and between PEG 400 and Me-β-CD, were found to be statistically significant ($p<0.0001$) (see Figure 11). It can be assumed that the inclusion and non-inclusion interactions occurring between the FH and CD are governed by a binding mechanism whose equilibrium enables the release of the FH. Only the non-complexed form of the drug can cross the membrane with MW cut-off of 1000 Da. Furthermore, in the absence of Me-β-CD, neither effect of PEG 400 nor any possible interaction with the two other excipients were found to contribute to diffusion. This assumption is based on the comparison of F3 (3% HPMC E50) and F5 (1% HPMC E50) profiles with those of F1 and F9, respectively, which contain an additional 3% of PEG 400 ($p= 0.130$ for F1 vs F3, and $p=0.104$ for F5 vs F9). However, a negative effect of PEG 400 can be hypothesized when Me-β-CD is incorporated in the synthesis, as indicated by the experimental values of F2 and F4, that both contain 6% Me-β-CD.

Compounds with high lipophilicity and low aqueous solubility are often formulated into liquid suspensions to mitigate significant fluctuations in the administered dose, which can arise due to the variability in the volume of nasal secretions. However, it is important to note that these formulations are primarily intended for local action, as a significant portion of the volume is often swallowed. Hence, solid formulations are preferred for brain targeting. In this context, the diffusion profiles shown in Figure 1 demonstrate that a CD-based film composition can significantly enhance FH permeation compared to FH solution. Specifically, negligible drug levels were found in the receptor compartment of the Franz cells after FH solution loading. According to FDA, FH is classified as a BCS Class II drug, indicating an anticipated favorable permeability through the nasal epithelium. However, the limited volume of nasal fluids exacerbates the challenge posed by the inadequate water solubility of these molecules, emerging as the primary limiting step for successful permeation. Consequently, an inclination towards exhibiting characteristics akin to a Class IV drug is observed. The maximum FH permeation across the nasal mucosa, applying nasal film technology, was found to be equal to $10.4 \pm 0.159$ % of the loading dose, while the FH solution reached the value of $2.91 \pm 0.403$ %. The more favorable permeation profile is observed by the F3 formulation containing as sole excipient 3% of HPMC E50 (see Figure 2). However, in the case of formulation F4, an increasing trend is observed, that becomes significantly higher from the time point of 90 min onwards (p<0.01), reaching a value of $5.21 \pm 0.428$ % at the time point of 120 min.

The comparison of F3 performance in vitro and ex vivo suggests that the compound diffuses more efficiently across the biological membrane than through the artificial one. Therefore, it can be hypothesized that the mucoadhesion of HPMC E50, optimal at pH=6, favors the direct interaction between the lipophilic drug and the mucosa. In the case of F4, which incorporates an additional 6% of Me-$\beta$-CD in its composition, the interweaving of HPMC molecules within the CD-drug network can serve as release modulator. This enables the sustained release of the drug, as evidenced by the permeation profile of this formulation across the nasal mucosa. Mucosa retention data of each formulation expressed as the percent (%) of the loading dose retained by the cellulose membrane or nasal mucosa $\pm$ SD, are included in Table 9.

The comparison of F3 performance in vitro and ex vivo suggests that the compound diffuses more efficiently across the biological membrane than through the artificial one. In the case of F4, which incorporates an additional 6% of Me-$\beta$-CD in its composition, the interweaving of HPMC molecules within the CD-drug network can serve as release modulator. This enables the sustained release of the drug, as evidenced by the permeation profile of this formulation across the nasal mucosa. At the low level of HPMC E50 (1% w/w), the presence of PEG 400 at its high level (3% w/w) was found to exhibit a negative effect on FH permeation. The profile of F9 revealed that a negligible amount of drug was detected in the receptor compartment at all the time points of the study (see Figure 2). Mucosa retention data of each formulation expressed as the percent (%) of the loading dose retained by the cellulose membrane $\pm$ SD, are included in Table 9.

**Table 9.** Percent (%) of the FH loading dose retained by the cellulose membrane and the nasal mucosa barrier, of the formulations F1-F7, F9-F11 (mean +/- SD, n= 3).

| Formulation | % of the dose retained by the cellulose membrane $\pm$ SD | % of the dose retained by the nasal mucosa barrier $\pm$ SD |
|---|---|---|
| $F_1$ | $1.3 \pm 0.07$ | $2.2 \pm 0.78$ |
| $F_2$ | $2.7 \pm 0.17$ | $6.8 \pm 1.34$ |
| $F_3$ | $1.1 \pm 0.17$ | $1.6 \pm 0.42$ |
| $F_4$ | $3.6 \pm 1.36$ | $5.3 \pm 3.3$ |
| $F_5$ | $1.1 \pm 0.40$ | $1.1 \pm 0.00$ |
| $F_6$ | $2.5 \pm 0.15$ | $10.0 \pm 2.42$ |
| $F_7$ | $2.0 \pm 0.11$ | $7.6 \pm 0.73$ |
| $F_9$ | $1.9 \pm 0.35$ | $3.1 \pm 0.34$ |
| $F_{10}$ | $2.1 \pm 0.42$ | $9.2 \pm 2.07$ |
| $F_{11}$ | $2.4 \pm 0.55$ | $8.9 \pm 1.94$ |

The results of formulation F8 are not presented as its composition does not allow the formation of film able to be detached from the blister.

**Example 7**

**Pharmacokinetic profiles of FH after intranasal and per os administration**

FH, also known by the trade name Gilenya®, is a medication approved for the treatment of MS. Although it is characterized by efficient absorption and has a 93% oral bioavailability, it undergoes extensive hepatic metabolism. In this regard, IN administration constitutes an alternative that ensures efficient NTB and systemic delivery with lower administered dose, reducing the exposure of peripheral organs, bypassing the first pass effect from liver, and achieving a significant enhancement of the available dose fraction at the target site. The preclinical data in literature is essentially limited, and the available studies lack a detailed description of experimental protocol applied, in terms of exact administration method and tissue collection procedures.

In this study, the oral administration of FH in mice using the gavage method resulted in a serum $C_{max}$ value of 0.28 $\pm$ 0.086 $\mu$g/mL at the time point of 10 h, while a smaller initial peak (0.21 $\pm$ 0.013 $\mu$g/mL) is observed at the early time point of 15 min (see Figure 3). The FDA Clinical and Pharmacology review reports that a single oral dose of fingolimod in humans' results in an absorption shoulder observed approximately 2 h after administration, followed by a $C_{max}$ value at the time point of 8 to 12h (Center for Drug Evaluation and Research [CDER]. Application number: 22-527. Clinical pharmacology and biopharmaceutics review(s) [online]. Available from URL: https://www.accessdata.fda.gov/drugsatf da_docs/nda/2010/022527orig1s000clinpharmr.pdf).

Fingolimod is characterized by a very slow absorption when given PO, reaching a wide plateau phase between 12 and 36 h. Generally, after a single-dose administration, the maximum concentration is reported in various times depending on the animal model (4 to 12 h), or on the population characteristics in the context of clinical studies (8 to 36 h). Additionally, the impact of precipitation and subsequent redissolution processes should be considered due to the weakly base character of fingolimod and its extremely low solubility in intestinal pH conditions (<0.01 mg/mL in pH 6.8, at both 25°C and 37°C). In cases where substantial precipitation happens in the gastrointestinal tract prior to the drug being adequately absorbed, it may lead to a decreased absorption rate and suboptimal systemic exposure. In contrast, the PK profiles of formulations F3 and F4 suggest that direct drug transfer to the bloodstream is achieved via nasal administration, leading to higher $C_{max}$ values compared to the oral solution (p>0.05). At the time points of 15 and 45 min, the $C_{max}$ values for F3 and F4 were found to be 0.35 $\pm$ 0.051 and 0.38 $\pm$ 0.065 $\mu$g/mL, respectively (see Figure 3). It is important to note that the administered dose in the case of IN formulations was 2.5 times lower than the oral one. The systemic absorption of low molecular weight molecules (MW< 900-1000 Da) can be mainly occurred on the respiratory area of the nasal cavity. At this site, the high permeability and vascularization of mucosa allow for fast systemic absorption approaching the $T_{max}$ value reported after the FH intravenous administration in rats.

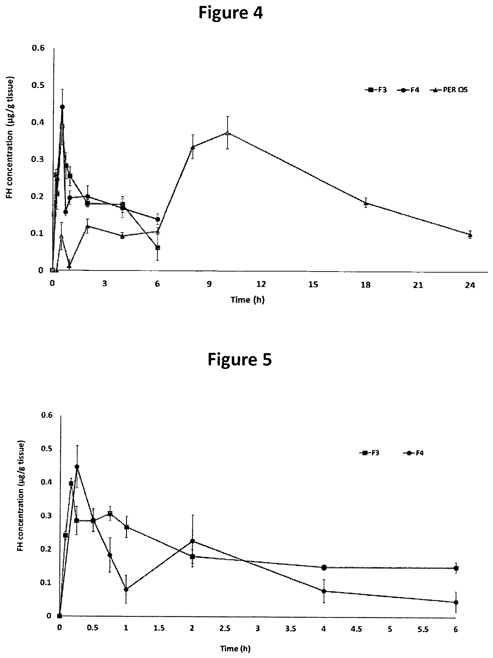

The comparison between formulations F3 and F4 revealed that the incorporation of Me-$\beta$-CD delays the FH absorption from the respiratory epithelium of nasal cavity. Nevertheless, this delay does not affect the extent of FH systemic exposure (see Figure 3). On the other hand, higher $C_{max}$ levels are reported in brain with the F4 formulation (p<0.05), indicating a positive effect of Me-$\beta$-CD that leads to more efficient brain targeting (0.39 $\pm$ 0.12 and 0.44 $\pm$ 0.11 $\mu$g/mL for F3 and F4, respectively, at the time point of 30 min) (see Figure 4). A possible explanation for these observations lies in the pattern of interaction of Me-$\beta$-CD with the respiratory and olfactory mucosa due to their different cell composition. Me-$\beta$-CD can increase the permeability in PC12 cell monolayers, which act as neuron-like cells, but does not affect permeation across Caco-2-monolayers, chosen as epithelial-like cells. Furthermore, the CD influences the drug's interaction with the mucus and its release from the formulation, resulting in a lag time of 30 min in reaching the maximum systemic concentration (see Figure 3).

The serum and brain profiles after PO administration are similar, with a delay observed at the smaller initial peak in brain (0.12 $\pm$ 0.043 $\mu$g/mL, 2 h after administration), as anticipated due to the slow absorption. The $C_{max}$ value was reported at the time point of 10 h and found to be equal to 0.37 $\pm$ 0.088 $\mu$g/mL. The brain concentration-time profiles for the two nasal formulations suggest that the presence of CD influences FH release, probably due to the interactions developed between the drug and CD molecules (see Figure 4). It can be postulated that the first concentration peak observed at 30 min, in the case of F4, corresponds to the drug's free fraction, which has dissolved in nasal fluids and is promptly absorbed into the brain. The second peak, with $C_{max}$ at 2 h, implies a slower release from the formulation or the contribution of trigeminal nerve in transportation. The double-peak phenomenon was mostly ascribed to the pathway of trigeminal nerve, which requires a longer time to be traveled. However, the presence of Me-$\beta$-CD in both cases probably suggests that CD-drug complexation is also responsible for the slower absorption of a dose fraction in both formulation cases.

The IN administration of FH has been performed at a preclinical level using rats with lysolecithin (LPC)-induced demyelination. Particularly, the IN administration of FH saline drops for two weeks resulted in approximately 3 times higher levels in brain than the oral administration of the same dose. The efficient and fast delivery of FH to the brain within the first 30 min, through nasal film formulations, can be considered a mode of acceleration in reaching the brain steady state concentration (Css). Once-daily oral dosing of FH, ranging from 0.125 to 5 mg/day, results in achieving steady state in 1 to 2 months. Simultaneous administration of FH nasal film with possibly lower oral dose than the usual could allow for adequate and prompt achievement of Css condition, and reduction of side effects. Although the discontinuation rate of fingolimod is not considered high (13.6 %), concerns arise, as several cases have reported unexpected rebound/reactivation of the disease after drug cessation. The most frequently cited adverse event leading to treatment discontinuation was the elevated liver transaminases. Therefore, the lower hepatic drug exposure after IN administration can be considered as an additional benefit that reduces the risk of liver damage.

The measured levels of FH in the olfactory bulb provided substantial evidence confirming that direct NTB delivery of the drug occurs after nasal films administration (see Figure 5). Both formulations, F3 and F4, showed similar $C_{max}$ values (0.40 $\pm$ 0.036 and 0.48 $\pm$ 0.0.14 $\mu$g/mL) at 10 and 15 min after administration. The double-peak observed in brain concentration profile of the F4 formulation is also noticeable in the olfactory profile which further supports the possibility of CD-drug interactions contributing to the appearance of the second peak.

## Example 8

### Serum FH Non-compartmental pharmacokinetic analysis

The NCA sparse methodology was utilized to calculate all serum FH pharmacokinetic parameters, which are listed in Table 10. The AUC values obtained after IN administration of nasal films were found to be 4 and 2 times higher, in the case of F3 and F4 respectively, compared to the respective time interval (6 h) after FH oral administration [$AUC_{0-6h}$ of 0.72 (h × μg)/mL]. As anticipated, the PO $AUC_{0-t}$ (t=24h) and $AUC_{inf}$ were higher than the respective $AUC_{0-t}$ (t=6h) and $AUC_{inf}$ values of both IN formulations, due to the longer sampling period and the different absorption mechanism and kinetics. The $C_{max}$ in serum showed a 25% and 36% increase with nasal administration of F3 and F4, respectively (Table 10). In addition, the values of % $F_{rel}$, calculated using $AUC_{inf}$ values, equal to 48.3% and 66.9% for F3 and F4, respectively, reveal that F4 formulation perform better for systemic FH absorption after nasal administration compared to F3 formulation. To assess the performance of nasal films in comparison to the oral solution more accurately, the % $F_{rel\,(0-6h)}$ values were calculated.

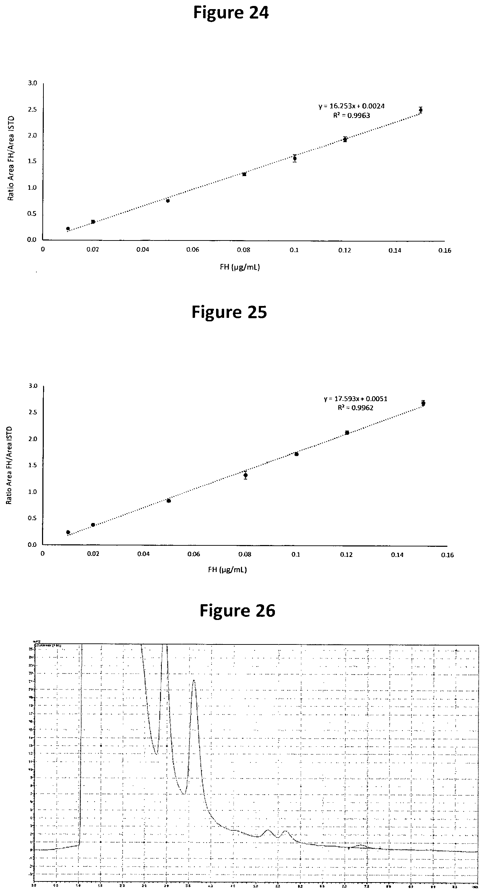

The determined values of 295% and 343%, for F3 and F4, respectively, demonstrate the efficiency of both formulations, as well as of the administration route, in enhancing the early FH systemic exposure (Table 10). Limited available preclinical studies do not allow for a direct comparison of the PK parameters within the same animal model. Particularly, to the best of our knowledge this study represents the first PK study of fingolimod administered via the nasal route, while sparse animal studies on oral delivery were found in the literature, which described the application of an analytical method in a rat PK study where 3 mg/kg of fingolimod were given orally and fingolimod oral administration in rats and dogs in different doses (2.8 and 7.5 mg/kg for rats, and 3 mg/kg for dogs). However, none of these studies specified the method used for FH oral administration, such as intragastric gavage, administration via drinking water or food, or using a feeding needle. In general, these studies reported lower $C_{max}$ and $T_{max}$ values. Nonetheless, due to the lack of detailed reference on the sampling schedule, particularly during the absorption phase (<10h), and the absence of information on the administration method used, further comparisons cannot be made. The predicted human clearance resulted by the application of allometric scaling using the equation [$CL_{human}=CL_{animal}\times (BW_{human}/BW_{animal})^b$] in F3, F4, and oral solution data (with the allometry exponent fixed at 0.8 described), revealed the values of 6.24, 4.53, and 2.88 L/h, respectively. These values are close to the clearance values of healthy volunteers reported in literature (6.3 ± 2.3 L/h) (Gilenya, INN-fingolimod, Summary of products characteristics, EMA https://www.ema.europa.eu/en/documents/product-information/gilenya-epar-product-information_en.pdf) The difference in $t_{1/2}$ values observed between the two modes of administration can be attributed to the distinct terminal slopes, λ, evident in the log-transformed FH serum concentration vs. time plots (see Figure 25). This variation arises from the disparate sampling schedules, with a duration of up to 6 h and 24 h for IN and PO administration respectively. As a result, the % AUC extrapolation to infinity is calculated to be 8.98% for F3, 24.6% for F4, and 23.4 % for oral administration. In both cases, the terminal slope was estimated using linear regression analysis on the log-transformed concentration data vs. time, employing the last three data points.

**Table 10.** Non-compartmental analysis on serum data using sparse data methodology.

| Pharmacokinetic Parameters | FH (% RSE) | | |
|---|---|---|---|
| | F3 | F4 | Per os |
| $AUC_{0-t}$(h × μg)/mL * | 0.86 (0.039) | 1.0 (0.055) | 3.8 (0.27) |
| $C_{max}$ (μg/mL) | 0.35 (0.021) | 0.38 (0.029) | 0.28 (0.038) |
| $AUC_{inf}$(h × μg)/mL | 0.96 | 1.3 | 5.0 |
| $T_{max}$(h) | 0.25 | 0.75 | 10 |
| $t_{1/2}$(h) | 1.63 | 2.95 | 8.75 |
| $CL_S$ (mL/h) | 10.9 | 7.92 | 5.03 |
| % $F_{rel}$(0-6h) | 48.3 [*295] | 66.9 [*343] | - |

*t=6h for F3, F4, and t=24h for Per os administration; Fingolimod Hydrochloride (FH); Area under the curve (AUC); clearance from serum (CLs) calculated as Dose/AUC and scaled by 1/F, where F is FH serum absolute bioavailability; % $F_{rel}$ IN vs per os, calculated using $AUC_{inf}$ and $AUC_{(0-6h)}$ up to 6 h after administration (0-6h).

## Example 9

### Brain FH Non-compartmental pharmacokinetic analysis

The NCA sparse methodology was employed to calculate all the brain PK parameters of FH listed in Table 11. The AUC values obtained from nasal film administration were found to be 2 times higher for both nasal films (F3, F4) compared to the corresponding time interval (6 h) after FH oral administration [$AUC_{0-t}$ (t=6h) of 0.52 (h $\times$ pg)/mL]. As expected, the PO $AUC_{0-t}$ (t=24h) and for oral administration were higher than the respective values for both IN formulations due to the longer sampling period and the different absorption mechanisms and kinetics. The $C_{max}$ in the brain after nasal films administration was found to be the same as for the oral solution, despite the half dose used in their case (see Table 11). The values of % $F_{rel\ (0-6h)}$ for F3 and F4, equal to 519% and 534%, respectively, show that both IN formulations managed to significantly increase the brain concentration of FH at early time after administration. Additionally, the values of % $F_{rel}$, calculated using $AUC_{inf}$ values, were 72.9% and 113% for F3 and F4, respectively, indicating that the F4 formulation performed better for NTB FH delivery compared to F3. In literature, the superiority of FH nasal delivery has been demonstrated only in repeated IN administration of 0.3 mg/kg for 14 days. Moreover, in a previous study, only two brain drug measurements are reported after the single oral dose of 7.5 mg/kg in rats, while full serum and brain PK FH profile after single oral dose of 10 mg/kg in dogs is provided. The difference in $t_{1/2}$ values observed between the two modes of administration can be attributed to the distinct terminal slopes, $\lambda$, evident in the log-transformed FH brain concentration vs. time plots (see Figure 26). This variation arises from the different sampling schedules, with durations of up to 6 hours and 24 hours for IN and PO administration, respectively. Consequently, the % AUC extrapolation to infinity is calculated to be 37.1% and 58.3% for F3 and F4, respectively, and 18.7% for oral administration. In both cases, the terminal slope was estimated by applying linear regression analysis to the log-transformed concentration data vs. time, considering the last three data points. However, it is important to note that the calculated $AUC_{inf}$ value in the case of F4, based on the terminal slope, should be considered with caution due to the high value of % AUC extrapolation.

**Table 11.** Non-compartmental analysis on total brain using sparse data methodology

| Pharmacokinetic Parameters | FH (% RSE) | | |
| --- | --- | --- | --- |
| | F3 | F4 | Per os |
| $AUC_{0-t}$ (h $\times$ $\mu$g)/g* | 1.1 (0.062) | 1.1 (0.071) | 4.8 (0.23) |
| $C_{max}$ ($\mu$g/g) | 0.39 (0.050) | 0.44 (0.048) | 0.37 (0.039) |
| $AUC_{inf}$ (h $\times$ $\mu$g)/g | 1.7 | 2.7 | 5.9 |
| $T_{max}$ (h) | 0.5 | 0.5 | 10 |
| $t_{1/2}$ (h) | 6.99 | 7.71 | 7.49 |
| $CL_B$ (g/h) | 6.09 | 3.93 | 4.24 |
| % $F_{rel}$ (0-6h) | 72.9 [*519] | 113 [*534] | - |

*t=6h for F3, F4, and t=24h for Per os administration; Fingolimod Hydrochloride (FH); Area under the curve (AUC); clearance from brain ($CL_B$) calculated as Dose/AUC and scaled by 1/F, where F is FH brain absolute bioavailability; % $F_{rel}$ IN vs per os, calculated using $AUC_{inf}$ and $AUC_{(0-6h)}$ up to 6 h after administration (0-6h).

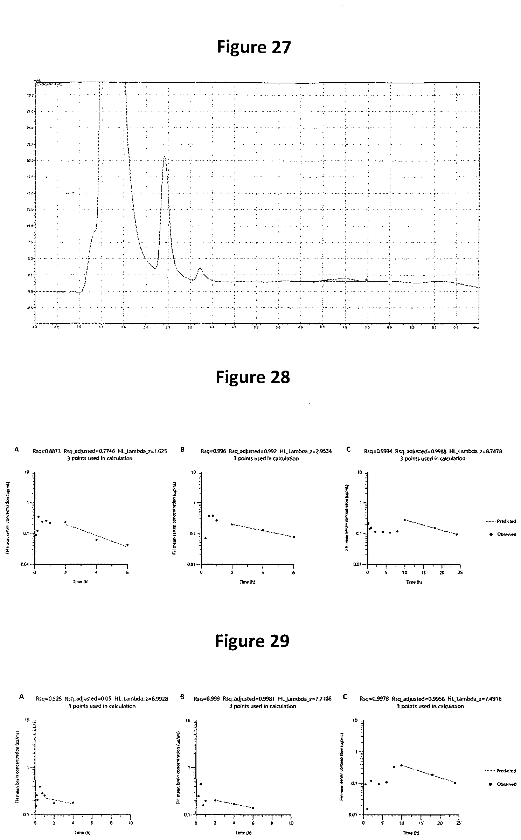

The log-transformed FH serum and brain concentration vs. time plots are presented in Figure 28.

**Example 10**

**Data analysis by nose-to-brain delivery indexes**

FH presents dose-proportional exposure in both single and multiple doses ranging from 0.125 to 5 mg. Hence, linear pharmacokinetics can be assumed and the relative % DTE and % DTP indexes can be applied to quantitatively analyze brain drug delivery following IN administration. The % $DTE_{rel}$ assesses the extent of drug delivery to the brain after IN administration compared to the PO administration, while the % $DTP_{rel}$ index measures the contribution of direct IN delivery to brain transport relative to the amount delivered via the systemic circulation. Both formulations show %DTE values higher than 100 (176 % and 156% for F3 and F4, respectively), confirming the superiority of IN route in FH brain targeting. Furthermore, the positive values of %DTP (43.3 % and 35.8% for F3 and F4, respectively) suggest that a significant portion of the IN dose directly entered the brain through the olfactory and/or trigeminal nerve pathways, in addition to the amount that reached the brain through systemic circulation. The values of indexes, especially % $DTP_{rel\ (0-6\ h)}$, suggest a more efficient brain targeting in the case of F3, opposite to the result revealed by the comparison of % $F_{rel}$ values, but consistent with the results obtained from ex vivo experiments. However, it is important to note the high % AUC extrapolation to infinity (58%) observed in the case of F4, causing possible inaccuracy in the calculated $AUC_{inf}$ value, and is probably attributed to the delayed brain absorption due to the presence of Me-$\beta$-CD, which allows for a sustained release of the drug, providing a flattened profile after the 1-hour time point.

Fingolimod hydrochloride (FH) has emerged as a vital medication for managing Multiple Sclerosis (MS). Despite its high oral bioavailability of 93%, it is plagued by slow oral absorption ($T_{max}$=8-12 h) and extensive hepatic metabolism. Intranasal administration has emerged as an alternative to address these limitations, ensuring efficient central nervous system delivery and minimizing peripheral exposure and first-pass metabolism.

This study aimed to develop and evaluate FH nasal films for enhanced drug delivery.

A Design of Experiments approach was employed to formulate FH nasal films, utilizing HPMC E50 as a film-forming polymer, PEG 400 as a plasticizer, and Me-$\beta$-CD as a permeation enhancer. Two formulations with superior in vitro and ex vivo performance were selected for in vivo evaluation. A comparative pharmacokinetic study was conducted in C57BL/6J mice in the brain and serum after administration of nasal films and oral FH solution, respectively. Sparse sampling and non-compartmental analysis were used.

Intranasal (IN) delivery offers several advantages in brain targeting, allowing for direct transport to the CNS, resulting in higher levels of drug concentration in the brain. Enhanced drug levels in the brain can be achieved through the anatomical connection between the nasal cavity and the CNS via the olfactory and trigeminal nerves. The involvement of the trigeminal nerve in innervating both the respiratory and olfactory regions highlights the potential significance of these areas in NTB delivery [9]. The respiratory region comprises a large area of the nasal cavity (80-90%), while the olfactory region is located on the roof of each nostril (<10% of the total surface). One key advantage of IN delivery is the bypassing of first-pass metabolism. In particular, the intestinal and hepatic metabolism often results in drug degradation or alteration of its structure, reducing the available dose fraction that can produce pharmacological activity.

In this context, the formulation of FH in a nasal dosage form was considered a promising approach for more efficient brain targeting, avoiding the effect of first pass hepatic metabolism, and possibly accelerating the achievement of steady state in blood and serum. For this purpose, the recently developed technology of nasal film was implemented, applying DoE. The design included the same factors/components at their low and high levels, and the resulting films underwent in vitro and ex vivo characterization. Two possible syntheses of FH nasal film, used for the comparative PK study with the oral FH solution, in C57BL/6J mice. Brain and serum distribution pharmacokinetics of the drug were studied applying the sparse sampling NCA with Phoenix® 8.3.5 (Certara, https://www.certara.com/software/phoenix-winnonlin/, Princeton, NJ, USA) software. The relative IN bioavailability ($F_{rel}$) and relative Drug Targeting Efficiency Percentage ($DTE_{rel}$) and nose-to-brain Direct Transport Percentage ($DTP_{rel}$) indexes were also calculated.

FH nasal films efficiently delivered the drug to both serum ($C_{max(F3)}$=0.35 $\pm$ 0.021, $C_{max(F4)}$=0.38 $\pm$ 0.029 $\mu$g/mL) and brain ($C_{max(F3)}$=0.39 $\pm$ 0.05, $C_{max(F4)}$=0.44 $\pm$ 0.048 $\mu$g/mL), achieving higher levels than oral delivery. Brain relative bioavailability (% Frel, 0-6h) was 519% and 534%, while serum % Frel (0-6h) was 295% and 343%.

The rapid nose-to-brain delivery within 30 minutes, in contrast to 10-hour Tmax of the oral solution, indicates the potential of a combined IN and oral treatment regimen. This approach could expedite the attainment of steady-state concentrations, offering a promising method for managing multiple sclerosis (MS).

The application of nasal film technology to a lipophilic molecule with low aqueous solubility for the development of a NTB delivery system demonstrated efficient and rapid drug delivery to both serum and brain. Higher drug levels were observed at these sites compared to oral delivery during the first 6 hours after administration. As a result, nasal films effectively enhance the relative bioavailability and early exposure of FH in the brain and serum, even when administering a dose that is 2.5 times lower. The presence of Me-$\beta$-CD in the film's synthesis did not affect the extent of exposure in either bloodstream or brain. However, it can be hypothesized that it delays the absorption from both sites, resulting in higher $T_{max}$ in the serum and sustained drug release in the brain. Overall, achieving $C_{max}$ within the first 30 min after nasal film administration, in contrast to the longer time required for oral solution, underscores the potential clinical application of a combined IN and oral treatment, to reach the Css more quickly.

**Claims**

1. A sphingosine-1-phosphate receptor modulator for use in the treatment of a disorder in a subject, the use comprising nasal administration of the sphingosine-1-phosphate receptor modulator to the subject.

2. A sphingosine-1-phosphate receptor modulator for use according to claim 1, wherein the disorder is multiple sclerosis.

3. A sphingosine-1-phosphate receptor modulator for use according to any one of claim 1 or 2, wherein the sphingosine-1-phosphate receptor modulator is selected from fingolimod, ozanimod, siponimod, ponesimod, etrasimod, ceralifimod, zectivimod, and cenerimod.

4. A sphingosine-1-phosphate receptor modulator for use according to any one of claims 1-3, wherein the sphingosine-1-phosphate receptor modulator is fingolimod.

5. A sphingosine-1-phosphate receptor modulator for use according to any one of claims 1-4, wherein the sphingosine-1-phosphate receptor modulator is a composition comprising the sphingosine-1-phosphate receptor modulator.

6. A sphingosine-1-phosphate receptor modulator for use according to claim 5, wherein the composition comprises the sphingosine-1-phosphate receptor modulator in an amount of 0.05 - 0.10 %w/w relative to the weight of the composition.

7. A sphingosine-1-phosphate receptor modulator for use according to any one of claim 5 or 6, wherein the composition comprises the sphingosine-1-phosphate receptor modulator and a film-forming polymer.

8. A sphingosine-1-phosphate receptor modulator for use according to claim 7, wherein the film-forming polymer is a cellulose or a derivative thereof.

9. A sphingosine-1-phosphate receptor modulator for use according to any one of claim 7 or 8, wherein the composition comprises the film-forming polymer in an amount of 0.5 - 5.0 %w/w relative to the weight of the composition.

10. A sphingosine-1-phosphate receptor modulator for use according to any one of claims 5-9, wherein the composition further comprises a permeation enhancer.

11. A sphingosine-1-phosphate receptor modulator for use according to claim 10, wherein the permeation enhancer is a cyclodextrin (CD) or derivative thereof.

12. A sphingosine-1-phosphate receptor modulator for use according to any one of claim 10 or 11, wherein the composition comprises the permeation enhancer in an amount of 0.5 - 10.0 %w/w relative to the weight of the composition.

13. A sphingosine-1-phosphate receptor modulator for use according to any one of claims 5-12, wherein the composition further comprises a plasticizer.

14. A sphingosine-1-phosphate receptor modulator for use according to claim 13, wherein the plasticizer is a polyethylene glycol.

15. A sphingosine-1-phosphate receptor modulator for use according to any one of claim 13 or 14, wherein the composition comprises the plasticizer in an amount of 0.5 - 5.0 %w/w relative to the weight of the composition.

## Figure 1

## Figure 2

## Figure 3

## Figure 4

## Figure 5

## Figure 6

## Figure 7

## Figure 8

y = 59076x - 6356.7
$R^2$ = 0.9999

FH µg/mL

## Figure 9

## Figure 10

## Figure 11

## Figure 12

## Figure 13

## Figure 14

## Figure 15

## Figure 16

## Figure 17

## Figure 18

## Figure 19

## Figure 20

## Figure 21

## Figure 22

## Figure 23

## Figure 24

$y = 16.253x + 0.0024$
$R^2 = 0.9963$

## Figure 25

$y = 17.593x + 0.0051$
$R^2 = 0.9962$

## Figure 26

## Figure 27

## Figure 28

## Figure 29

Europäisches
Patentamt

European
Patent Office

Office européen
des brevets

## EUROPEAN SEARCH REPORT

**Application Number**

EP 24 38 6119

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GHASEMI-KASMAN MARYAM ET AL: "Intranasal administration of fingolimod (FTY720) attenuates demyelination area in lysolecithin-induced demyelination model of rat optic chiasm", MULTIPLE SCLEROSIS AND RELATED DISORDERS, [Online] vol. 59, 10 January 2022 (2022-01-10), page 103518, XP093260923, NL ISSN: 2211-0348, DOI: 10.1016/j.msard.2022.103518 Retrieved from the Internet: URL:https://doi.org/10.1016/j.msard.2022.103518> [retrieved on 2025-03-19] | 1-6 | INV. A61K31/137 A61K9/00 A61K47/10 A61K47/38 A61K47/40 A61P25/28 A61K31/397 A61K31/405 A61K31/4245 A61K31/426 A61K31/4439 A61K31/454 |
| Y | * abstract * * page 1, left-hand column - page 2, left-hand column * * page 4, left-hand column - page 7, left-hand column * ----- | 7-15 | |
| X | CN 118 593 457 A (UNIV ZHENGZHOU) 6 September 2024 (2024-09-06) * claims 1-5 * * paragraphs [0001], [0005] - [0025] * ----- | 1,3-5 | TECHNICAL FIELDS SEARCHED (IPC) A61K A61P |
| X | US 9 592 208 B2 (RANE SUPRIYA [US]; NOVARTIS AG [CH]) 14 March 2017 (2017-03-14) * claims 1-11 * * column 1, line 11 - line 38 * * column 2, line 49 - column 3, line 9 * * column 3, line 62 - column 6, line 11 * * column 7, line 24 - line 47 * ----- -/-- | 1,3-15 | |

The present search report has been drawn up for all claims

| Place of search | Date of completion of the search | Examiner |
|---|---|---|
| The Hague | 26 March 2025 | De Masi, Alessia |

EPO FORM 1503 03.82 (P04C01)

page 1 of 2

Europäisches
Patentamt

European
Patent Office

Office européen
des brevets

## EUROPEAN SEARCH REPORT

**Application Number**

EP 24 38 6119

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | PAPAKYRIAKOPOULOU PARASKEVI ET AL: "Development and In Vitro-Ex Vivo Evaluation of Novel Polymeric Nasal Donepezil Films for Potential Use in Alzheimer's Disease Using Experimental Design", PHARMACEUTICS, vol. 14, no. 8, 21 August 2022 (2022-08-21), page 1742, XP093262299, Switzerland ISSN: 1999-4923, DOI: 10.3390/pharmaceutics14081742 * abstract * * page 1 - page 3 * * page 5 - page 6 * * page 10 - page 12 * * page 15 - page 18 * * tables 1,2 * * figures 4,6 * | 7-15 | |
| | ----- | | TECHNICAL FIELDS SEARCHED (IPC) |
| Y,D | BALAFAS EG, PAPAKYRIAKOPOULOU PI, KOSTOMITSOPOULOS NG, VALSAMI GN: "Intranasal Administration of a Polymeric Biodegradable Film to C57BL/6 Mice", J Am Assoc Lab Anim Sci., vol. 62, no. 2 2023, pages 179-184, XP093261137, Retrieved from the Internet: URL:https://doi.org/10.30802/aalas-jaalas-22-000091 * abstract * * page 179, left-hand column - page 180, left-hand column * * page 181, right-hand column - page 183, right-hand column * | 7-15 | |
| | ----- | | |

The present search report has been drawn up for all claims

| Place of search | Date of completion of the search | Examiner |
|---|---|---|
| The Hague | 26 March 2025 | De Masi, Alessia |

EPO FORM 1503 03.82 (P04C01)

**page 2 of 2**

## ANNEX TO THE EUROPEAN SEARCH REPORT
## ON EUROPEAN PATENT APPLICATION NO.

EP 24 38 6119

This annex lists the patent family members relating to the patent documents cited in the above-mentioned European search report.
The members are as contained in the European Patent Office EDP file on
The European Patent Office is in no way liable for these particulars which are merely given for the purpose of information.

26-03-2025

| Patent document cited in search report | | | Publication date | Patent family member(s) | | | Publication date |
|---|---|---|---|---|---|---|---|
| CN 118593457 | A | | 06-09-2024 | NONE | | | |
| US 9592208 | B2 | | 14-03-2017 | AR | 085749 | A1 | 23-10-2013 |
| | | | | AU | 2012236357 | A1 | 10-10-2013 |
| | | | | BR | 112013024430 | A2 | 20-12-2016 |
| | | | | CA | 2831600 | A1 | 04-10-2012 |
| | | | | CL | 2013002810 | A1 | 14-02-2014 |
| | | | | CN | 103476400 | A | 25-12-2013 |
| | | | | CO | 6771459 | A2 | 15-10-2013 |
| | | | | CY | 1118423 | T1 | 28-06-2017 |
| | | | | CY | 1122868 | T1 | 05-05-2021 |
| | | | | DK | 2694037 | T3 | 16-01-2017 |
| | | | | DK | 3143990 | T3 | 02-03-2020 |
| | | | | EA | 201391442 | A1 | 30-04-2014 |
| | | | | EA | 201790436 | A2 | 31-10-2017 |
| | | | | EC | SP13012912 | A | 29-11-2013 |
| | | | | EP | 2694037 | A1 | 12-02-2014 |
| | | | | EP | 3143990 | A1 | 22-03-2017 |
| | | | | ES | 2610966 | T3 | 04-05-2017 |
| | | | | ES | 2773482 | T3 | 13-07-2020 |
| | | | | GT | 201300227 | A | 05-05-2015 |
| | | | | HK | 1190309 | A1 | 04-07-2014 |
| | | | | HR | P20170021 | T1 | 10-03-2017 |
| | | | | HR | P20200249 | T1 | 15-05-2020 |
| | | | | HU | E031286 | T2 | 28-06-2017 |
| | | | | JO | 3177 | B1 | 08-03-2018 |
| | | | | JP | 6019101 | B2 | 02-11-2016 |
| | | | | JP | 2014509652 | A | 21-04-2014 |
| | | | | KR | 20140014194 | A | 05-02-2014 |
| | | | | LT | 2694037 | T | 10-01-2017 |
| | | | | LT | 3143990 | T | 25-02-2020 |
| | | | | MA | 34981 | B1 | 01-03-2014 |
| | | | | MX | 342522 | B | 03-10-2016 |
| | | | | MY | 163746 | A | 31-10-2017 |
| | | | | MY | 175633 | A | 02-07-2020 |
| | | | | NZ | 615023 | A | 29-07-2016 |
| | | | | PE | 20140162 | A1 | 12-02-2014 |
| | | | | PE | 20170913 | A1 | 12-07-2017 |
| | | | | PH | 12013501953 | A1 | 25-11-2023 |
| | | | | PL | 2694037 | T3 | 31-05-2017 |
| | | | | PL | 3143990 | T3 | 18-05-2020 |
| | | | | PT | 2694037 | T | 17-01-2017 |
| | | | | PT | 3143990 | T | 04-03-2020 |
| | | | | SG | 193256 | A1 | 30-10-2013 |
| | | | | SI | 3143990 | T1 | 31-03-2020 |
| | | | | TW | 201244711 | A | 16-11-2012 |

EPO FORM P0459

For more details about this annex : see Official Journal of the European Patent Office, No. 12/82

page 1 of 2

## ANNEX TO THE EUROPEAN SEARCH REPORT
## ON EUROPEAN PATENT APPLICATION NO.

EP 24 38 6119

This annex lists the patent family members relating to the patent documents cited in the above-mentioned European search report.
The members are as contained in the European Patent Office EDP file on
The European Patent Office is in no way liable for these particulars which are merely given for the purpose of information.

26-03-2025

| Patent document cited in search report | Publication date | Patent family member(s) | | Publication date |
|---|---|---|---|---|
| | | US | 2014371323 A1 | 18-12-2014 |
| | | US | 2017135967 A1 | 18-05-2017 |
| | | US | 2018193291 A1 | 12-07-2018 |
| | | US | 2019247335 A1 | 15-08-2019 |
| | | US | 2020276137 A1 | 03-09-2020 |
| | | WO | 2012135561 A1 | 04-10-2012 |
| | | ZA | 201306636 B | 28-05-2014 |

EPO FORM P0459

For more details about this annex : see Official Journal of the European Patent Office, No. 12/82

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SOURI E ; ZARGARPOOR M ; MOTTAGHI S ; AHMADKHANIHA R ; KEBRIAEEZADEH A.** A Stability-Indicating HPLC Method for the Determination of Fingolimod in Pharmaceutical Dosage Forms.. *Sci Pharm.*, 2014, vol. 83 (1), 85-93
- **BALAFAS EG ; PAPAKYRIAKOPOULOU PI ; KOSTOMITSOPOULOS NG ; VALSAMI GN.** Intranasal Administration of a Polymeric Biodegradable Film to C57BL/6 Mice. *J Am Assoc Lab Anim Sci.*, 2023, vol. 62 (2), 179-184
- **PAPAKYRIAKOPOULOU P ; BALAFAS E ; COLOMBO C ; REKKAS DM ; KOSTOMITSOPOULOS N ; VALSAMI G.** Nose-to-Brain delivery of donepezil hydrochloride following administration of an HPMC-Me-β-CD-PEG400 nasal film in mice. *J. Drug Deliv Sci Technol.*, 2023, vol. 84, 104463
- **GAGE GJ ; KIPKE DR ; SHAIN W.** Whole animal perfusion fixation for rodents. *J Vis Exp*, 2012, vol. 65, 3564
- **SOURI E ; ZARGARPOOR M ; MOTTAGHI S ; AHMADKHANIHA R ; KEBRIAEEZADEH A.** A Stability-Indicating HPLC Method for the Determination of Fingolimod in Pharmaceutical Dosage Forms. *Sci Pharm.*, 2014, vol. 83 (1), 85-93
- **TIOZZO FASIOLO L ; MANNIELLO MD ; BANELLA S ; NAPOLI L ; BORTOLOTTI F ; QUARTA E ; COLOMBO P ; BALAFAS E ; KOSTOMITSOPOULOS N ; REKKAS DM.** Flurbiprofen sodium microparticles and soft pellets for nose-to-brain delivery: Serum and brain levels in rats after nasal insufflation. *Int J Pharm.*, 2021, vol. 605, 120827
- **PAPAKYRIAKOPOULOU P ; REKKAS DM ; COLOMBO G ; VALSAMI G.** Development and In Vitro-Ex Vivo Evaluation of Novel Polymeric Nasal Donepezil Films for Potential Use in Alzheimer's Disease Using Experimental Design. *Pharmaceutics.*, 2022, vol. 14 (8), 1742
- **BALAFAS EG ; PAPAKYRIAKOPOULOU PI ; KOSTOMITSOPOULOS NG ; VALSAMI GN.** Intranasal Administration of a Polymeric Biodegradable Film to C57BL/6 Mice. *J Am Assoc Lab Anim Sci.*, 2023, vol. 62 (2), 179-184
- **SOURI E ; ZARGARPOOR M ; MOTTAGHI S ; AHMADKHANIHA R ; KEBRIAEEZADEH A**. A Stability-Indicating HPLC Method for the Determination of Fingolimod in Pharmaceutical Dosage Forms.. *Sci Pharm.*, 2014, vol. 83 (1), 85-93
- **EUROPEAN MEDICINES AGENCY**. Guideline on bioanalytical method validation. European Medicines Agency, 2023